(19) **Europäisches Patentamt / European Patent Office / Office européen des brevets**

(11) **EP 3 984 946 A1**

(12) **EUROPEAN PATENT APPLICATION**

(43) Date of publication:
**20.04.2022 Bulletin 2022/16**

(21) Application number: **21189669.1**

(22) Date of filing: **04.08.2021**

(51) International Patent Classification (IPC):
**B82B 3/00** (2006.01)   **B82Y 5/00** (2011.01)
**B82Y 15/00** (2011.01)   **C07K 7/06** (2006.01)
**C12N 5/0786** (2010.01)   **C12N 5/0775** (2010.01)

(52) Cooperative Patent Classification (CPC):
**B82Y 5/00; B82Y 15/00; C07K 7/06; C12N 5/0645; C12N 5/0662**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.10.2020 KR 20200131888**
**13.10.2020 KR 20200131889**

(71) Applicant: **Korea University Research and Business Foundation**
**Seoul 02841 (KR)**

(72) Inventors:
• **KIM, Young-Keun**
  **06009 Seoul (KR)**
• **KANG, Heemin**
  **06344 Seoul (KR)**
• **KO, Min-Jun**
  **07974 Seoul (KR)**
• **BAE, Gun-Hyu**
  **18477 Gyeonggi-do (KR)**
• **MIN, Sun-Hong**
  **16380 Gyeonggi-do (KR)**

(74) Representative: **Zech, Stefan Markus**
**Meissner Bolte Patentanwälte**
**Rechtsanwälte Partnerschaft mbB**
**Postfach 86 06 24**
**81633 München (DE)**

(54) **NANOHELIX-SUBSTRATE COMPLEX FOR CONTROLLING BEHAVIOR OF REGENERATIVE CELLS, PREPARING METHOD THEREOF, AND METHOD OF CONTORLLING BEHAVIOR OF REGENERATIVE CELLS BY USING THE SAME**

(57) The present invention relates to a nanohelix-substrate complex for controlling behavior of regenerative cells, a preparing method thereof, and a method of controlling behavior of regenerative cells by using the nanohelix-substrate complex, and the method of controlling behavior of regenerative cells may efficiently control cell adhesion and phenotypic polarization of macrophages ex vivo or in vivo and cell adhesion and differentiation of stem cells ex vivo or in vivo by controlling application/non-application of a magnetic field to the nanohelix-substrate complex.

[FIG. 1]

## Description

### TECHNICAL FIELD

The present invention relates to a nanohelix-substrate complex for controlling behavior of regenerative cells, a preparing method thereof, and a method of controlling behavior of regenerative cells by using the nanohelix-substrate complex, and particularly, to a method of controlling behavior of regenerative cells according to application/non-application of a magnetic field to the nanohelix-substrate complex, in which the regenerative cell is a macrophage or a stem cell.

### BACKGROUND ART

A macrophage is the main cell responsible for innate immunity. Most of the macrophages are fixed in the whole body, but some of the macrophages are present in the form of monocytes in the blood. The monocytes may be divided into dendritic cells or macrophages. Most of the macrophages are fixed, representatively include dust cells, microglial cells, Kupffer cells, and Langerhans cells, and the like, and the macrophages are distributed throughout the body, and when antigens invade, the macrophages eat the antigens or secrete toxins to destroy and remove the antigens, and deliver antigens to lymphocytes and trigger an immune response. When an enemy invades the wound, the monocytes in the blood go out of the blood vessels like neutrophils and are divided into macrophages to remove bacteria. Further, the macrophages are divided into a free form which moves to various places in the body and performs phagocytosis, and a fixed form which is fixed to designated organs and performs phagocytosis. The macrophages in the fixed form include liver Kupffer cells, alveolar macrophages, connective tissue structure (histiocyte), and brain microglia cells, and the like.

As a method of efficiently controlling the regeneration and anti-inflammatory effects of macrophages, a technology by the presentation of ligands in vivo is used. However, there is a problem in that the existing micro-scale integrin-coupled ligand peptide (RGD) uncaging controls the adhesion of host macrophages, but does not control the functional phenotypic polarization of macrophages.

Accordingly, the applicant of the present invention had developed the technology for controlling adhesion and polarization of macrophages by controlling periodicity and sequences of nanobarcode ligands and filed the technology for a patent application.

In addition, the applicant of the present application intends to propose a technology that is capable of providing a more improved and bio-friendly technology compared to the previously filed macrophage adhesion and polarization control technology below, and particularly, intends to propose a technology that is capable of changing a characteristic of cells in real time by using external stimuli after injection, rather than a method of designing and inserting ligands in advance.

Stem cells can proliferate through self-renewal, and have the potential to differentiate into various cells, such as bone, fat, muscle, myocardium, blood vessels, and cartilage. Recently, in order to regenerate damaged tissues and organs by using these characteristics, many studies have been conducted on transplantation of stem cells or cells differentiated from stem cells. In addition, biomaterials that can help stem cells to differentiate into specific cells are also being actively studied.

As a method of efficiently controlling the regenerative effect of stem cells, a technology through the presentation of ligand in vivo is used. However, there is a problem in that the existing micro-scale integrin-coupled ligand peptide (RGD) uncaging controls the adhesion of host stem cells, but does not control the differentiation of stem cells.

In this respect, the present applicant developed the technology of controlling adhesion and differentiation of stem cells by controlling periodicity and sequences of nanobarcode ligands and filed the technology for a patent application.

In addition, the applicant of the present application intends to propose a technology that is capable of providing a more improved and bio-friendly technology compared to the previously filed stem cell adhesion and differentiation control technology below, and particularly, intends to propose a technology that is capable of changing a characteristic of cells in real time by using external stimuli after injection, rather than a method of designing and inserting ligands in advance.

[Prior Art Literature]

(Patent Document) Korean Patent No. 10-1916588

### SUMMARY OF THE INVENTION

The present invention is conceived to solve the foregoing problems, and is to provide a substrate including ligand-coated nanohelices, and a method of controlling behavior of regenerative cells by controlling an application of a magnetic field to the ligand-coated nanohelices.

In particular, the method of controlling the regenerative cells according to the present invention relates to a method of controlling cell adhesion and polarization of macrophages and controlling cell adhesion and differentiation of stem cells.

The present invention provides a nanohelix-substrate complex for controlling behavior of regenerative cells, including: a substrate; one or more nanohelices chemically coupled to the substrate; and one or more integrin-coupled ligand peptides chemically coupled to the nanohelix, in which the nanohelix is formed of a spiral nanowire and includes one or more metal elements, the nanohelix has a length of 100 nm to 20 $\mu$m, and the nanohelix has a length reversibly changed depending on application/non-application of a magnetic field within a range of Equation 1 below.

[Equation 1]

$$|L_1 - L_0| > 10 \text{ nm}$$

In Equation 1, $L_1$ is a length of the nanohelix when the magnetic field is applied, and $L_0$ is a length of the nanohelix when the magnetic field is not applied.

Further, the present invention provides a method of controlling behavior of regenerative cells, the method including: preparing a nanohelix by electrodepositing a solution including one or more metal elements; coupling a carboxylate substituent to the nanohelix by mixing the nanohelix and a first suspension; manufacturing a substrate coupled with the nanohelix by soaking a substrate of which a surface is activated in a solution containing the nanohelix to which the carboxylate is coupled; coupling a linker to a distal end of the nanohelix by soaking the substrate coupled with the nanohelix in a solution containing a polyethylene glycol linker; and coupling an integrin-coupled ligand peptide (RGD) to the nanohelix by mixing a second suspension containing the integrin-coupled ligand peptide (RGD) and the activated substrate coupled with the nanohelix.

Another exemplary embodiment of the present invention includes controlling behavior of regenerative cells by treating the nanohelix-substrate complex for controlling cell adhesion and polarization of the macrophages by applying a magnetic field in a range from 20 mT to 7 T, and the nanohelix has a length reversibly changed within Equation 1 below depending on application/non-application of the magnetic field.

[Equation 1]

$$|L_1 - L_0| > 10 \text{ nm}$$

In Equation 1, $L_1$ is a length of the nanohelix when the magnetic field is applied, and $L_0$ is a length of the nanohelix when the magnetic field is not applied.

The nanohelix-substrate complex for controlling behavior of regenerative cells according to the present invention is capable of reversibly controlling cell adhesion and polarization by controlling application/non-application of a magnetic field to the nanohelix coated with the integrin-coupled ligand peptide, and efficiently controlling cell adhesion and phenotypic polarization of macrophages and cell adhesion and differentiation of stem cells in vivo or ex vivo.

## BRIEF DESCRIPTION OF THE DRAWINGS

FIG. 1 is a schematic diagram illustrating a nanohelix-substrate complex for controlling behavior of regenerative cells and a method of controlling adhesion and polarization of macrophages by using the same according to an exemplary embodiment of the present invention.

FIG. 2 is a scanning electron microscope image of a nanohelix according to the present invention.

FIG. 3 is a High-Angle Annular Dark Field Scanning Transmission Electron Microscope (HAADF-STEM) image, a Scanning Electron Microscope (SEM) image, an Energy Dispersive Spectroscopy (EDS) mapping image, and a High-Resolution Scanning Transmission Electron Microscopy (HR-STEM) image of the nanohelix according to the present invention, and a scale bar of the HAADF-STEM and the EDS represent 250 nm, a scale bar of the SEM represents 1 $\mu$m, and a scale bar of the HR-STEM represents 4Å.

FIG. 4 is a graph (a) illustrating an EDS analysis result and a graph (b) illustrating an EELS analysis result of the nanohelix according to the present invention.

FIG. 5 is a graph illustrating a vibrating-sample magnetometry measurement result of the nanohelix according to the present invention.

FIG. 6 is an X-ray diffraction analysis graph of the nanohelix according to the present invention.

FIG. 7 is a High-Resolution Transmission Electron Microscopy (HRTEM) image of the nanohelix according to the present invention, the left scale bar represents 300 nm, and the right scale bar represents 2 nm.

FIG. 8 is an image schematically illustrating an operation of preparing a nanohelix-substrate complex according to the present invention.

FIG. 9 is a diagram illustrating a result of a Fourier Transform Infrared Spectroscopy (FT-IR) analysis of the nanohelix-substrate complex according to the present invention.

FIGS. 10 and 11 are Atomic Force Microscope (AFM) images of the nanohelix according to the present invention, and a scale bar represents 500 nm.

FIG. 12 is a confocal immunofluorescent image (a) of F-actin, nuclei, and vinculin in macrophages cultured (after 24 hours) by using the nanohelix-substrate complex according to the present invention, and a graph (b) illustrating a cell density, a cell area, and a cell elongation factor calculated based on the result of the confocal immunofluorescent experiment, and a scale bar represents 20 $\mu$m.

FIG. 13 is a confocal immunofluorescent image (a) of live cells and dead cells in macrophages cultured (after 24 hours) by using the nanohelix-substrate complex according to the present invention, and a graph (b) illustrating cell viability calculated based on the result of the confocal immunofluorescent experiment, and a scale bar represents 20 $\mu$m.

FIG. 14 is a diagram illustrating a result of an adhesion experiment of macrophages for bimodal switching in a substrate with no nanohelix or the nanohelix-substrate complex in which integrin ligand (RGD) is not coupled according to a comparative example of the present invention.

FIG. 15 is a confocal immunofluorescent image of F-actin, nuclei, and vinculin in macrophages cultured for 36 hours by regulating application of a magnetic field at an interval of 12 hours by using the nanohelix-substrate complex according to the present invention, and a scale bar represents 20 $\mu$m.

FIG. 16 is a result of an experiment of the control of macrophage adhesion-dependent phenotypic polarization by application of a magnetic field by using the nanohelix-substrate complex according to the present invention, and a scale bar represents 20 $\mu$m.

FIG. 17 is a result of an experiment for controlling application of a magnetic field when there is no stimulation medium matched to a polarization phenotype (that is, M1 expression in an M2-stimulation medium or M2 expression in an M1-stimulation medium) by using the nanohelix-substrate complex according to the present invention.

FIG. 18 is a confocal immunofluorescent image (a) of ROCK2 and nuclei after macrophages are cultured in an M1 or M2 medium under bimodal switching of stretching ("ON) in which a magnetic field is applied and compression ("OFF") in which a magnetic field is not applied for 36 hours, and a graph (b) illustrating ROCK2 immunofluorescent intensity calculated based on a result of the confocal immunofluorescent experiment, and a scale bar represents 20 $\mu$m.

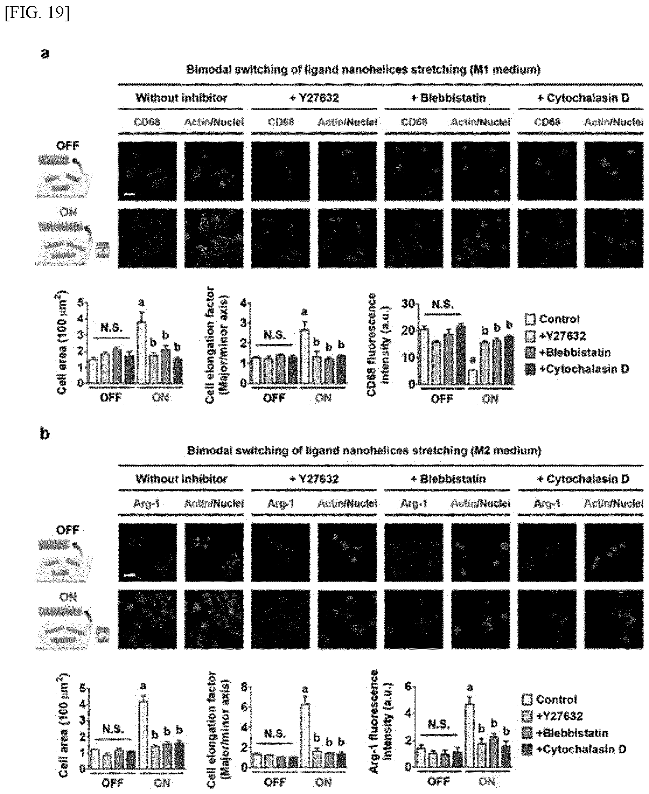

FIG. 19 is a diagram illustrating an experiment using the nanohelix-substrate complex according to the exemplary embodiment of the present invention, and a of FIG. 19 is a confocal immunofluorescent image of Arg-1, and F-actin, and nuclei in macrophages cultured in an M1 polarization medium with and without an inhibitor for ROCK(Y27632), myosin II (blebbistatin), or actin polymerization (cytochalasin D) for 36 hours and a graph representing a cell area, a cell elongation factor, and CD68 fluorescence intensity calculated based on the confocal immunofluorescent experiment result, and b of FIG. 19 is a confocal immunofluorescent image of Arg-1, and F-actin, and nuclei in macrophages cultured in an M2 polarization medium with and without an inhibitor for ROCK(Y27632), myosin II (blebbistatin), or actin polymerization (cytochalasin D) and a graph representing a cell area, a cell elongation factor, and Arg-1 fluorescence intensity calculated based on the confocal immunofluorescent experiment result.

FIGS. 20 and 21 are diagrams illustrating a result of an experiment for control of adhesion and phenotype of host macrophages in vivo by using the nanohelix-substrate complex according to the present invention.

FIG. 22 is a diagram illustrating a result of an experiment of adhesion in vivo of host neutrophil to the substrate by using the nanohelix-substrate complex according to the present invention.

FIG. 23 is a schematic diagram illustrating a nanohelix-substrate complex for controlling behavior of regenerative cells and a method of controlling adhesion and differentiation of stem cells by using the same according to an exemplary embodiment of the present invention.

FIG. 24 is an SEM image of the nanohelix according to the present invention.

FIG. 25 is a High-Angle Annular Dark Field Scanning Transmission Electron Microscope (HAADF-STEM) image, a Scanning Electron Microscope (SEM) image, an Energy Dispersive Spectroscopy (EDS) mapping image, and a High-Resolution Scanning Transmission Electron Microscopy (HR-STEM) image of the nanocoil according to the present invention, a scale bar of the HAADF-STEM represent 250 nm, a scale bar of the SEM represents 1 $\mu$m, and a scale bar of the HR-STEM represents 4Å.

FIG. 26 is a graph (a) illustrating an EDS analysis result, and a graph and a mapping image (b) illustrating an EELS analysis result of the nanohelix according to the present invention, and a scale bar of (b) represents 200 nm.

FIG. 27 is a High-Resolution Transmission Electron Microscopy (HRTEM) image of the nanohelix according to the present invention, , the left scale bar represents 300 nm, and the right scale bar represents 2 nm.

FIG. 28 is an X-ray diffraction analysis graph of the nanohelix according to the present invention

FIG. 29 is a graph illustrating a vibrating-sample magnetometry measurement result of the nanohelix according to the present invention.

FIG. 30 is an image schematically illustrating an operation of preparing a nanohelix-substrate complex according to the present invention.

FIG. 31 is a diagram illustrating an FT-IR analysis result of the nanohelix-substrate complex according to the present invention.

FIGS. 32 and 33 are an Atomic Force Microscope (AFM) image and a graph representing the length of the nanocoil according to the present invention and a scale bar represents 500 nm.

FIG. 34 is a confocal immunofluorescent image (a) of F-actin, nuclei, and vinculin in macrophages cultured (after 24 hours) by using the nanohelix-substrate complex according to the present invention, and a graph (b) illustrating a cell density, a cell area, and a cell elongation factor calculated based on the result of the confocal immunofluorescent experiment, and a scale bar represents 50 $\mu$m.

FIG. 35 is a confocal immunofluorescent image of F-actin, nuclei, and vinculin in stem cells cultured (after 54 hours) by changing an application of a magnetic field every 18 hours by using the nanohelix-substrate complex according to the present invention, and a scale bar represents 50 $\mu$m.

FIG. 36 is a graph illustrating an adherent cell density, a cell area, focal adherence number, and an aspect ratio (a ratio of major axis/minor axis) of the stem cells cultured by changing the application of a magnetic field at an interval of 18 hours by using the nanohelix-substrate complex according to the present invention calculated based on the result of the confocal immunofluorescent experiment.

FIG. 37 is a confocal immunofluorescent image (a) of live cells and dead cells in stem cells cultured (after 48 hours) by using the nanohelix-substrate complex according to the present invention, and a graph (b) illustrating cell viability calculated based on the result of the confocal immunofluorescent experiment, and a scale bar represents 50 $\mu$m.

FIG. 38 is a diagram illustrating a result of an experiment for adhesion of stem cells for bimodal switching in a substrate having no nanohelix or a nanohelix-substrate complex to which the integrin ligand (RGD) is not coupled according to a comparative example of the present invention.

FIG. 39 is a confocal immunofluorescent image (a) of F-actin, nuclei, and vinculin in stem cells cultured for 36 hours by adjusting an application of a magnetic field at an interval of 18 hours by using the nanohelix-substrate complex according to the present invention, and a graph (b) illustrating nuclear/cytoplasmic YAP ratio calculated based on the result of the confocal immunofluorescent experiment, and a scale bar represents 50 $\mu$m.

FIG. 40 is a result of the confocal immunofluorescent analysis for F-actin, nuclei, and TAZ in stem cells cultured for 36 hours by adjusting an application of a magnetic field at an interval of 18 hours by using the nanohelix-substrate complex according to the present invention.

FIG. 41 is a result of the confocal immunofluorescent analysis for osteocalcin, F-actin, nuclei in stem cells cultured 5 days by using the nanohelix-substrate complex according to the present invention, in which an application of a magnetic field is adjusted at the second day.

FIG. 42 is a graph illustrating a quantitative analysis of the nuclear/cytoplasmic RUNX2 and ALP gene expression profile in stem cells cultured for 3 days by using the nanohelix-substrate complex according to the present invention, in which an application of a magnetic field is adjusted after one day.

FIG. 43 is a result of the confocal immunofluorescent analysis for ALP genes, RUNX2, F-actin, and nuclei in stem cells cultured 5 days by using the nanohelix-substrate complex according to the present invention, in which an application of a magnetic field is adjusted at the second day.

FIG. 44 is a result of the confocal immunofluorescent analysis for YAP, F-actin, and nuclei in stem cells cultured for 48 hours in a medium without an inhibitor and a medium with ROCK inhibitor (Y27632) and myosin II inhibitor (blebbistatin) by using the nanohelix-substrate complex according to the present invention.

FIG. 45 is a result of the confocal immunofluorescent analysis for YAP, F-actin, and nuclei in stem cells cultured for 48 hours in a medium without an inhibitor and a medium with actin polymerization inhibitor (cytochalasin D) by using the nanohelix-substrate complex according to the present invention.

FIG. 46 is a result of the confocal immunofluorescent analysis for TAZ, F-actin, and nuclei in stem cells cultured for 48 hours in a medium without an inhibitor and a medium with actin polymerization inhibitor (cytochalasin D), ROCK inhibitor (Y27632), and myosin II inhibitor (blebbistatin) by using the nanohelix-substrate complex according to the present invention.

FIG. 47 is a result of an experiment for host stem cell adhesion control in vivo by using the nanohelix-substrate complex according to the present invention.

FIG. 48 is a graph illustrating adherent cell density, cell area, focal adhesion number, aspect ratio (major axis/minor axis ratio), and nuclear/cytoplasmic YAP fluorescence ratio calculated from the confocal immunofluorescent image of FIG. 47.

## DETAILED DESCRIPTION

Hereinafter, in order to describe the present invention in more specifically, an exemplary embodiment of the present invention will be described in more detail with reference to the accompanying drawings. However, the present invention is not limited to the exemplary embodiment described herein, and may also be specified in other forms.

The present invention provides a nanohelix-substrate complex for controlling behavior of regenerative cells, the nanohelix-substrate complex including: a substrate; one or more nanohelices chemically coupled to the substrate; and one or more integrin-coupled ligand peptides chemically coupled to the nanohelix, in which the nanohelix is provided with a nanowire in a spiral form, includes one or more metal elements, has a length of 100 nm to 20 $\mu$m, and has a length reversibly changed depending on application/non-application of a magnetic field within a range of Equation 1 below.

## [Equation 1]

$$|L_1-L_0|>10 \text{ nm}$$

In Equation 1, $L_1$ is a length of the nanohelix when a magnetic field is applied, and $L_0$ is a length of the nanohelix when a magnetic field is not applied.

The "controlling the behavior of the regenerative cells" in the nanohelix-substrate complex for controlling the behavior of the regenerative cells according to the present invention means to control any one or more of cell adhesion, polarization, and differentiation of regenerative cells.

In particular, the regenerative cell is a stem cell or a macrophage, and the nanohelix-substrate complex for controlling the behavior of the regenerative cells may control cell adhesion and differentiation of stem cells or control cell adhesion and polarization of macrophages.

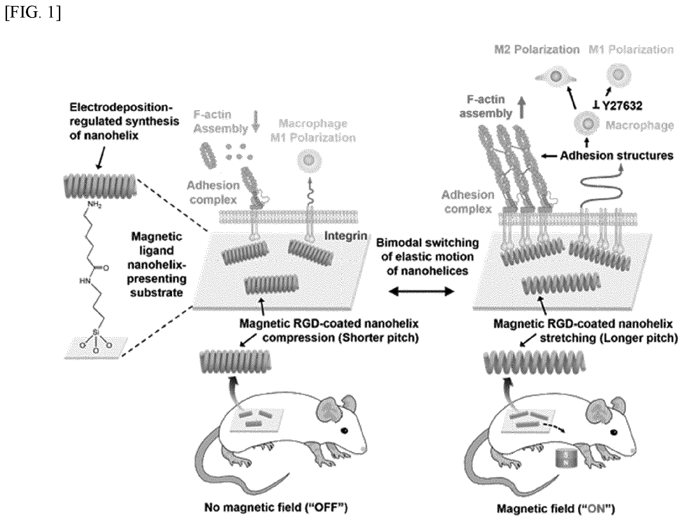

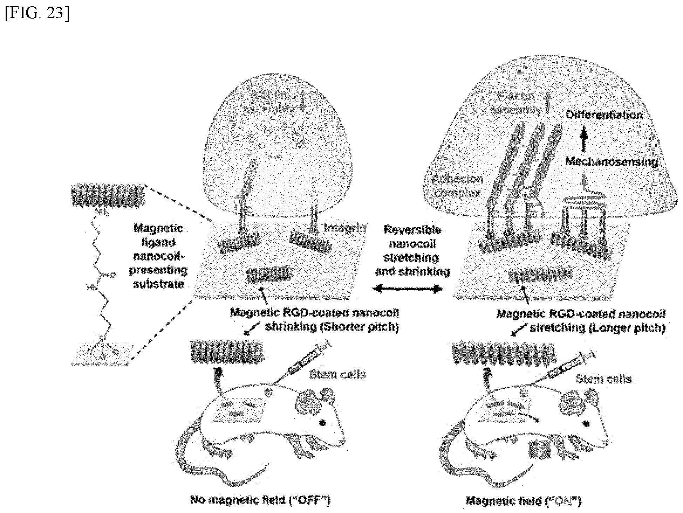

FIG. 1 is a schematic diagram illustrating a nanohelix-substrate complex for controlling behavior of regenerative cells and a method of controlling adhesion and polarization of macrophages by using the same according to the present invention, and FIG. 23 is a schematic diagram illustrating a nanohelix-substrate complex for controlling behavior of regenerative cells and a method of controlling adhesion and differentiation of stem cells by using the same according to present invention.

Referring to FIGS. 1 and 23, the nanohelix-substrate complex of the present invention includes: a substrate; one or more nanohelices chemically coupled to the substrate; and one or more integrin-coupled ligand peptides chemically coupled to the nanohelix, in which the nanohelix is provided with a nanowire in a spiral form and the nanowire includes one or more metal elements among cobalt (Co), iron (Fe), and nickel (Ni).

In particular, the nanohelix may be provided with a nanowire in a spiral form satisfying Equation 1.

## [Equation 1]

$$|L_1-L_0|>10 \text{ nm}$$

In Equation 1, $L_1$ is a length of the nanohelix when a magnetic field is applied, and $L_0$ is a length of the nanohelix when a magnetic field is not applied.

In Equation 1, the length of the nanohelix when the magnetic field is not applied may be 100 nm to 20 $\mu$m, 500 nm to 4 $\mu$m, or 1 $\mu$m to 3 $\mu$m.

As described above, when the magnetic field is applied, the nanohelix is stretched and has an increasing length, thereby promoting adhesion of stem cells in vivo. However, when the magnetic field is removed, the nanohelix is compressed, so that the length of the nanohelix returns to the existing length.

In particular, in Equation 1, the change in the length of the nanohelix depending on application/non-application of the magnetic field may be 10 nm or more, 20 nm or more, 10 nm to 500nm, or 10 nm to 100nm.

When the change in the length of the nanohelix in the nanohelix-substrate complex of the present invention does not satisfy Equation 1, the change in the length of the nanohelix is small, so there is no difference in cell adhesion, which is a problem.

An average length of a spiral outer diameter of the nanohelix may be 50 nm to 200 nm, or 100 nm to 200 nm. When the spiral outer diameter of the nanohelix is less than 100 nm, the nanohelix is too small, so that it is difficult for the integrin-coupled ligand peptide to be coupled at regular intervals, and when the spiral outer diameter of the nanohelix is larger than 200 nm, an area occupied by the nanohelix on the substrate is large, so that there is a problem in that it is difficult to distribute the nanohelices on the substrate at an appropriate density.

The nanohelix is formed of a nanowire, and the nanowire may include one or two or more metal elements among cobalt (Co), iron (Fe), and nickel (Ni), and the nanowire may be provided in the form of a wire having a circular cross-section, and has a diameter of 5 nm to 100 nm, 20 nm to 90 nm, or 60 nm to 90 nm. When the foregoing diameter of the wire is not satisfied, the nanohelices may not exhibit smooth stretching and compression.

The integrin-coupled ligand peptide coupled into the nanohelix may be a thiolated integrin-coupled ligand peptide, and the plurality of integrin-coupled ligand peptides is coupled to the nanohelix while being spaced apart from each other, and an average interval between the adjacent integrin-coupled ligand peptides may be 1 nm to 10 nm. When the average interval between the adjacent integrin-coupled ligand peptides is less than 1 nm, it is difficult to activate adhesion and polarization of macrophage even in the case where a magnetic field is applied, and when the average interval between the adjacent integrin-coupled ligand peptides is larger than 10 nm, adhesion and polarization of macrophage are activated even in the case where a magnetic field is not applied, so that there is a problem in that it is difficult to reversibly control the adhesion and polarization of macrophage by using the magnetic field.

When the magnetic field is applied to the nanohelix, the adjacent spirals in the nanohelix are spaced apart from each other, and a pitch between the adjacent spirals may be 1 nm to 100 nm, 1 nm to 50 nm, or 5 nm to 30 nm. In this case, when the magnetic field is applied, the pitch interval increases while the nanohelix is stretched. Accordingly, an interval between the integrin-coupled ligand peptides may also increase.

The integrin-coupled ligand peptide is the thiolated integrin-coupled ligand peptide, and a thiol group of the integrin-coupled ligand peptide may be coupled to the spiral nanohelices by a polyethylene glycol linker. The polyethylene glycol linker may be maleimide-poly(ethylene glycol)-NHS ester (Mal-PEG-NHS ester). The nanohelix includes the polyethylene glycol linker, so that coupling force between the nanohelix and the integrin-coupled ligand peptide increases to improve durability.

The nanohelix may have a structure in which carboxylate is coupled. The carboxylate substituent may be an amino acid derivate, in particular, aminocaproic acid. As described above, the nanohelix has the structure in which carboxylate is coupled, thereby increasing coupling force between the nanohelix and the substrate and the integrin-coupled ligand peptide.

The substrate is the substrate of which a surface is aminated, and may be the substrate, of which the surface is activated, by soaking the substrate in an aminosilane solution, and may have a structure in which the amino group on the surface of the substrate is coupled to a carboxyl group of the nanohelix through the EDC/NHS reaction.

Further, the substrate may be the substrate which is not coupled with the nanohelix and of which the surface is inactivated.

Further, the present invention provides a method of preparing a nanohelix-substrate complex for controlling behavior of regenerative cells, the method including: preparing a nanohelix by electrodepositing a solution containing one or more metal elements; coupling a carboxylate substituent to the nanohelix by mixing the nanohelix and a first suspension; manufacturing a substrate coupled with the nanohelix by soaking a substrate of which a surface is activated in a solution containing the nanohelix to which the carboxylate is coupled; coupling a linker to a distal end of the nanohelix by soaking the nanohelix-coupled substrate in a solution containing a polyethylene glycol linker; and coupling an integrin-coupled ligand peptide (RGD) to the nanohelix by mixing a second suspension containing the integrin-coupled ligand peptide (RGD) and the activated substrate coupled with the nanohelix.

In the preparing of the nanohelix, the solution containing the metal element may include one or two or more metal elements among cobalt (Co), iron (Fe), and nickel (Ni).

The preparing of the nanohelix includes: preparing a nano template including nano pores, and including a working electrode on one surface thereof; preparing a first metal precursor mixed solution containing a metal precursor solution containing ascorbic acid ($C_6H_8O_6$), vanadium (IV) oxide sulfate ($VOSO_4 \cdot xH_2O$), and a metal to be deposited; preparing a second metal precursor mixed solution by mixing the first metal precursor mixed solution and nitric acid ($HNO_3$); immersing the nano template in the second metal precursor mixed solution, and depositing metal nanohelices on the nano pores by an electrodepositing method by applying a current between a counter electrode and the working electrode inserted into the second metal precursor mixed solution; and selectively removing the working electrode and the nano template in the nano template on which the metal nanohelices are deposited.

As the nano template, an Anodic Aluminum Oxide (AAO) nanoframe, an inorganic nanoframe, or a polymer nanoframe is used. Herein, the case of using the AAO nanoframe is illustrated. A size of the nanowire is determined according to a diameter of a pore of the AAO nanoframe, and a length of the nanowire is determined according to a forming time and speed of the nanowire.

An average diameter of the nano pore may be 5 to 500 nm, 50 nm to 200 nm, or 100 nm to 200 nm.

The metal precursor solution may include at least one of cobalt sulfate (II) heptahydrate ($CoSO_4 \cdot 7H_2O$) and iron sulfate (II) heptahydrate ($FeSO_4 \cdot 7H_2O$).

A concentration of cobalt sulfate (II) heptahydrate ($CoSO_4 \cdot 7H_2O$) may be 30 mM to 100 mM, a concentration of vanadium(IV) oxide sulfate ($VOSO_4 \cdot xH_2O$) may be 30 mM to 100 mM, a concentration of iron sulfate(II) heptahydrate ($FeSO_4 \cdot 7H_2O$) may be 30 mM to 100 mM, and a concentration of ascorbic acid ($C_6H_8O_6$) may be 20 mM to 50 mM.

pH of the second mixed precursor mixed solution may be 1.5 to 2.5.

The method may further include immersing the nano template in the second metal precursor mixed solution and decompressing a plating bath containing the second metal precursor mixed solution. Pressure of the plating bath may be 100 Torr to 700 Torr.

A density of a current flowing in the working electrode during the electroplating may be 0.1 to 300 mA/cm$^2$, and an electroplating time may be one minute to 48 hours.

A silver (Ag) electrode layer having a thickness of 250 nm is formed on a bottom surface of the AAO nanoframe by an electron beam evaporation method. The electrode layer serves as a negative electrode during the electrodeposition. Herein, as the electrode layer, other metals or other conductive material layers may be used.

The coupling of the carboxylate substituent may be performed by mixing the nanohelix and the first suspension and reacting the nanohelix and the first suspension for 8 to 20 hours to 10 to 15 hours. The first suspension may contain an amino acid derivative containing a carboxylate substituent, and specifically, the amino acid derivative may be aminocaproic acid. The amino acid derivative may be coupled to the surface of the nanohelix by reacting the nanohelix with the first suspension.

The manufacturing of the substrate coupled with the nanohelix may be performed by soaking the substrate, of which the surface is activated, in the solution containing the nanohelix in which the carboxylate is coupled.

The substrate, of which the surface is activated, may be manufactured by immersing the substrate in the acidic solution containing any one or more of hydrochloric acid and sulfuric acid for 30 minutes to 2 hours or 30 minutes to 1 hour. Through this, the coupling with an amino group is facilitated by coupling a hydroxyl group to the surface of the substrate, thereby effectively performing activation of the surface of the substrate.

In the manufacturing of the substrate coupled with the nanohelix, the surface of the substrate may be aminated by soaking the substrate, of which the surface is activated, in the amino-silane solution under a dark condition. The amino-silane solution may include (3-aminopropyl)triephoxysilane (APTES). In this case, the amination of the surface of the substrate means that the amine group is coupled onto the substrate. The surface of the substrate is aminated by immersing the substrate in the amino-silane solution, so that the substrate may be coupled with the nanohelix through the EDC/NHS reaction.

The coupling of the linker to the distal end of the nanohelix may be performed by soaking the nanohelix-coupled substrate in the solution containing the polyethylene glycol linker. The polyethylene glycol linker may be maleimide-poly(ethylene glycol)-NHS ester (Mal-PEG-NHS ester). The nanohelix includes the polyethylene glycol linker, so that coupling force between the nanohelix and the integrin-coupled ligand peptide increases to improve durability.

The coupling of the integrin-coupled ligand peptide to the nanohelix may be performed by mixing a second suspension including the integrin-coupled ligand peptide (RGD) and the activated nanohelix-coupled substrate. The second suspension may include the thiolated integrin-coupled ligand peptide.

The method may further include soaking the nanohelix-coupled substrate in a solution including a polyethylene glycol derivative and inactivating the surface of the substrate that is not coupled with the nanohelix, after the coupling of the integrin-coupled ligand peptide to the nanohelix. The polyethylene glycol derivative may be methoxy-poly(ethylene glycol)-succinimidylcarboxymethyl ester.

Further, the present invention provides a method of controlling behavior of regenerative cells, the method including controlling behavior of regenerative cells by treating the nanohelix-substrate complex for controlling behavior of the regenerative cells with a culture medium and then applying a magnetic field in a range from 20 mT to 7 T, and in which a length of the nanohelix is reversibly changed depending on application/non-application of a magnetic field, and the length of the nanohelix satisfies Equation 1 below, and in the controlling of the regenerative cells, the regenerative cells are stem cells or macrophages, and in the case where the regenerative cell is the stem cell, the controlling of the regenerative cells includes controlling adhesion and differentiation of the stem cells, and in the case where the regenerative cell is the macrophage, the controlling of the regenerative cells includes controlling adhesion and polarization of the macrophages.

[Equation 1]

$$|L_1 - L_0| > 10 \text{ nm}$$

In Equation 1, $L_1$ is a length of the nanohelix when a magnetic field is applied, and $L_0$ is a length of the nanohelix when a magnetic field is not applied.

In the controlling of the cell adhesion and the polarization of the macrophages, it is possible to control adhesion and polarization of macrophages in vivo or ex vivo by reversibly changing the length of the nanohelix depending on application/non-application of the magnetic field to the nanohelix-substrate complex.

In particular, in the controlling of the adhesion and the polarization of the macrophages, when the magnetic field is not applied to the nanohelix-substrate complex, the nanohelix is compressed and a pitch interval of the nanohelix is decreased to promote an inflammatory (M1) phenotype.

Further, in the controlling of the cell adhesion and the polarization of the macrophages, when the magnetic field is applied to the nanohelix-substrate complex, the nanohelix is stretched and a pitch interval of the nanohelix is increased to promote a regenerative and anti-inflammatory (M2) phenotype.

For example, when the magnetic field is applied to the nanohelix-substrate complex and then the magnetic field is removed, the nanohelix is reversibly stretched and compressed.

Accordingly, it is possible to temporally and reversibly control the cell adhesion and polarization of macrophages by using the nanohelix-substrate complex according to the present invention.

The controlling of the cell adhesion and differentiation of the stem cells may control the cell adhesion and differentiation of the stem cells in vivo or ex vivo by reversibly changing the length of the nanohelix depending on application/non-application of a magnetic field to the nanohelix-substrate complex.

In particular, in the controlling of the adhesion and the differentiation of the stem cells, when the magnetic field is not applied to the nanohelix-substrate complex, the nanohelix is compressed and a pitch interval of the nanohelix is decreased to degrade adhesion and mechanosensing differentiation of stem cells.

Further, in the controlling of the cell adhesion and the differentiation of the stem cells, when the magnetic field is applied to the nanohelix-substrate complex, the nanohelix is stretched and a pitch interval of the nanohelix is increased to promote adhesion and mechanosensing differentiation of stem cells.

For example, when the magnetic field is applied to the nanohelix-substrate complex and then the magnetic field is removed, the nanohelix is reversibly stretched and compressed. In particular, when the magnetic field is applied to the nanohelix-substrate complex, the magnetic field is removed, and then the magnetic field is applied to the nanohelix-substrate complex again, the nanohelix may be stretched, compressed, and then stretched again.

Accordingly, it is possible to temporally and reversibly control the cell adhesion and differentiation of stem cells by using the nanohelix-substrate according to the present invention.

In particular, in Equation 1, it can be seen that a change in the length of the nanohelix depending on application/non-application of the magnetic field may be 10 nm or more, 20 nm or more, 10 nm to 500nm, or 10 nm to 100nm.

When the change in the length of the nanohelix in the nanohelix-substrate complex of the present invention does not satisfy Equation 1, the change in the length of the nanohelix is small, so there is no difference in cell adhesion performance, which is a problem.

Hereinafter, examples of the present invention will be described. However, the examples below are merely preferable examples of the present invention, and the scope of the present invention is not limited by the examples.

**[Preparation Example]**

**Preparation Example**

Prepare nanohelix

A nanohelix was prepared by using an AAO porous template having pores with 200 nm in diameter through electrodeposition. First, silver (Ag) was deposited on one surface of the AAO porous template by using an electronbeam evaporator. A metal ion precursor solution was prepared by mixing cobalt sulfate heptahydrate ($CoSO_4 \cdot 7H_2O$, 0.08M) and iron sulfate heptahydrate ($FeSO_4 \cdot 7H_2O$, 0.08M) in deionized water. In order to produce CoFe nanohelices, vanadium (IV) oxide sulfate ($VOSO_4 \cdot xH_2O$), and L-ascorbic acid (0.06 M) were added to the metal ion precursor solution. Next, nitric acid was then added to the precursor solution to adjust the pH to 2.5, the mixed precursor solution was injected into the pores of the AAO template pores, and then a current at constant current density of 20 mA/cm$^2$ was applied to deposit CoFe nanohelices. The nanotemplate was removed by reacting the CoFe nanohelix-deposited nanotemplate and 1 M of NaOH for 30 minutes at 45°C, followed by washing the CoFe nanohelix with deionized water to prepare the CoFe nanohelices. The washed CoFe nanohelices were suspended in 1 mL of deionized water before being coupled to the substrate.

**Comparative Preparation Example**

A nanohelix was prepared by the same method as that of Preparation Example 1 except that a negatively charged thiolated RGD peptide (CDDRGD, GL Biochem) was not added.

**[Example]**

**Example**

Prepare nanohelix-substrate complex

Aminocaproic acid was used to be coupled to a surface of a magnetic CoFe nanohelices based on an amine group that is reported to react with the native oxide layer of the nanohelices prepared in the preparation example. A mixed solution of 1 mL of nanohelices and 1 mL of 6 mM of an aminocaproic acid solution were stirred at a room temperature for 12 hours, and then centrifuged and washed with deionized water. A cell culture grade glass substrate (22 mm × 22 mm) was aminated to allow a carboxylate group on the surface of the nanohelix to be bonded to the amine group on the substrate. The substrate was first washed with a mixture in which hydrochloric acid and methanol were mixed at a ratio of 1:1 for 30 minutes and rinsed with deionized water. The washed substrate was activated in sulfuric acid for 1 hour and washed with deionized water. The substrate was aminated in 3-aminopropyl triethoxy silane (APTES) and ethanol (1:1) in a darkroom for 1 hour and washed with ethanol, followed by drying for 1 hour at 100°C. The aminocaproic acid-conjugated nanohelices were activated in 1 mL of deionized water containing 0.5 mL of 20 mM N-ethyl-N'-(3-(dimethylaminopropyl)carbodiimide) (EDC) and 0.5 mL of 20 mM N-hydroxysuccinimide (NHS) through EDC/NHS reaction for 3 hours, followed by washing with deionized water.

The aminated substrate was incubated with the activated nanohelices for 1 hour, followed by washing with deionized water. An integrin ligand was cultured in 1 mL of deionized water containing 0.04 mM of maleimide-poly(ethylene glycol)-NHS linker and 2 μ1 of N,N-Diisopropylethylamine (DIPEA, 2 μL) under the shaking in the dark for 16 hours, grafted on the surface of the substrate by mediating the amide bond formation, followed by washing with deionized water. To mediate the thiol-ene reaction, the substrate was cultured in 1 mL of deionized water containing thiolated RGD peptide ligands (GCGYGRGDSPG, GL Biochem, 0.04 M), 2 μL of N,N-diisopropylethylamine (DIPEA), and 10 mM Tris(2-carboxyethyl)phosphine hydrochloride (TCEP) for 2 hours in the dark, and then washed with deionized water. To minimize the non-RGD ligand specific macrophage adhesion before the culturing of the cell, the areas to which the nanohelix was not coupled were activated in 1 mL of deionized water containing 2 μL of N,N-diisopropylethylamine (DIPEA) and 100 μM methoxy-poly(ethylene glycol)-succinimidyl carboxymethyl ester in the dark for 2 hours, followed by washing to block the non-nanohelix-coated area of the substrate.

**Comparative Example 1**

A nanohelix-substrate complex was prepared by the same method except for using the prepared nanohelix Comparative Preparation Example 1.

**[Experimental Example]**

**Experimental Example 1**

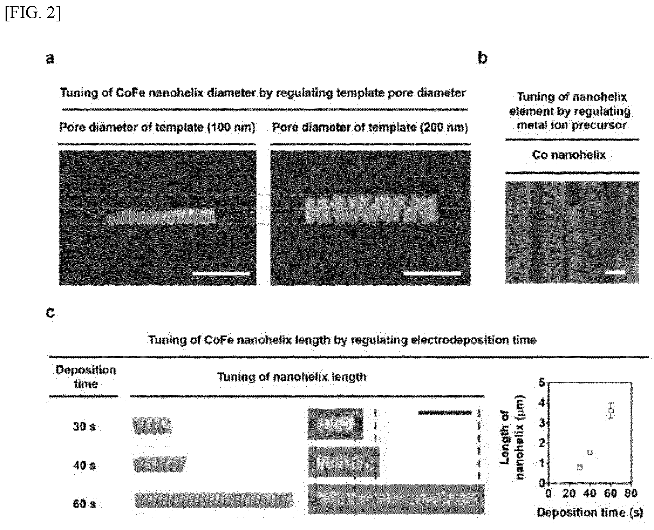

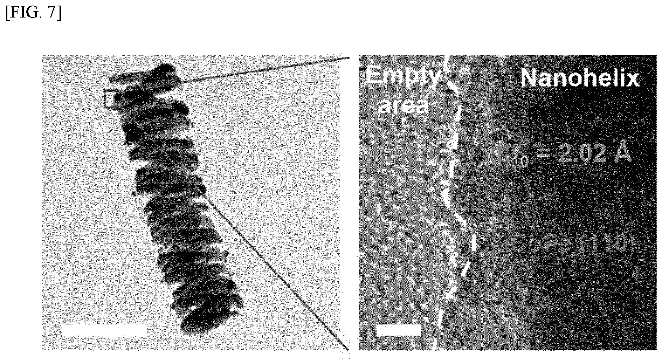

In order to check the form and the chemical characteristic of the nanohelix according to the present invention, the prepared nanohelices were photographed by using a Scanning Electron Microscope (SEM), a High-Angle Annular Dark-Field Scanning Transmission Electron Microscopy (HAADF-STEM), a High-Resolution Transmission Electron Microscope (HR-TEM), and a High-Resolution Scanning Transmission Electron Microscopy (HR-STEM), and then analyzed by using Energy Dispersive X-ray Spectroscopy (EDS), Electron Energy Loss Spectroscopy (EELS), Vibrating-Sample Magnetometry (VSM), and X-ray Diffraction (XRD), and a result thereof is represented in FIGS. 2 and 7.

FIG. 2 is an SEM image of the nanohelix according to the present invention. In particular, a of FIG. 2 is an SEM image of the CoFe nanohelix prepared by regulating a pore diameter of an electrodeposition template, b of FIG. 2 is an SEM image of cobalt nanohelices and the CoFe nanohelices, and c of FIG. 2 is an SEM image of the measured length of the CoFe nanohelix according to an electrodeposition time, and in this case, a scale bar represents 500 nm in a of FIG. 2, 200 nm in b of FIG. 2, and 1 $\mu$m in c of FIG. 2.

Referring to FIG. 2, according to the nanohelix according to the present invention, it can be seen that it is possible to regulate the diameter of the CoFe nanohelix according to the pore diameter of the electrodeposition template, it is possible to control a constituent element of the nanohelix according to the control of the metal ion precursor, and it is possible to regulate the length of the CoFe nanohelix according to an electrodeposition time.

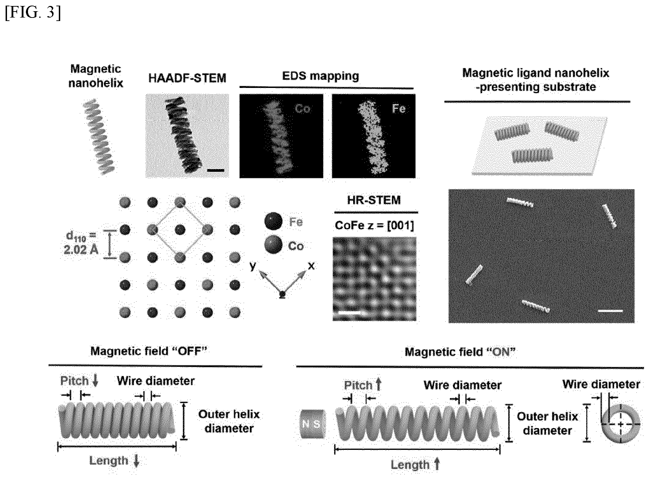

FIG. 3 is an HAADF-STEM image, an EDS mapping image, and an HR-STEM image of the nanohelix according to the present invention.

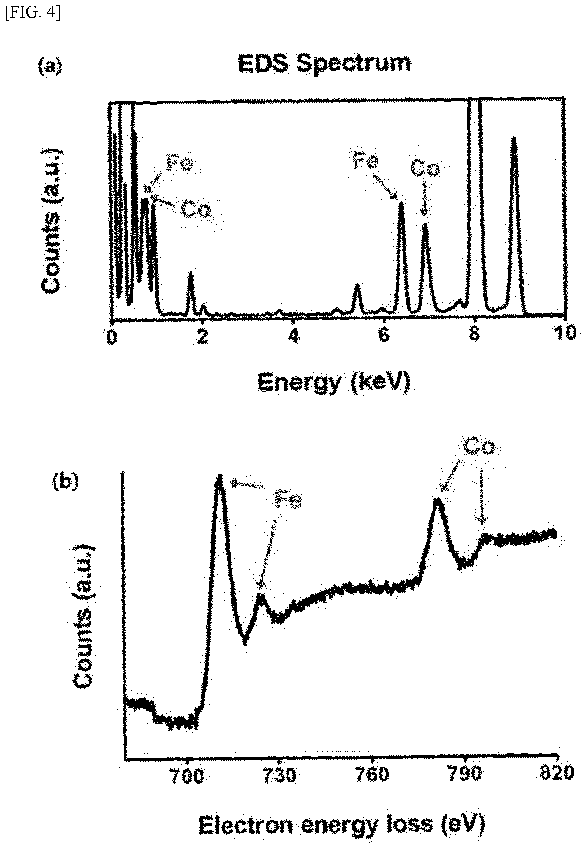

FIG. 4 is a graph (a) illustrating an EDS analysis result and a graph (b) illustrating an EELS analysis result of the nanohelix according to the present invention.

Referring to FIGS. 3 and 4, in the HAADF-STEM image, it can be seen that the nanohelix consists of cobalt (Co) and iron (Fe), each of which is constantly distributed with a distribution of about 50 atom%.

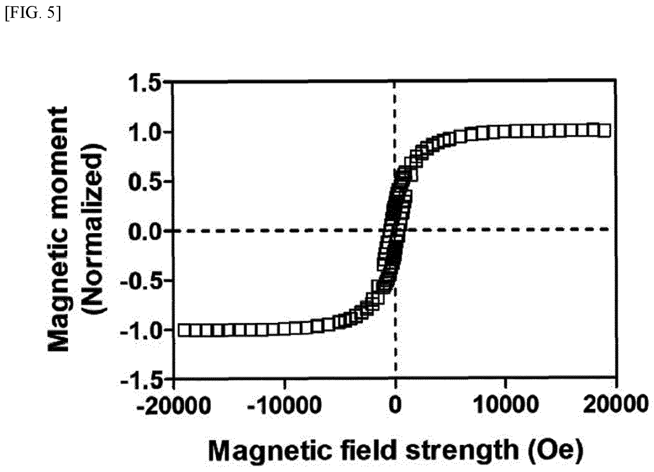

FIG. 5 is a graph illustrating a measurement result of a vibrating-sample magnetometry of the nanohelix according to the present invention. In particular, the magnetic characteristic of the nanohelix by cobalt and iron was confirmed, and through this, it can be recognized that reversible bimodal switching between nano stretching ("ON") and nano-compression ("OFF") of the nanohelix is possible.

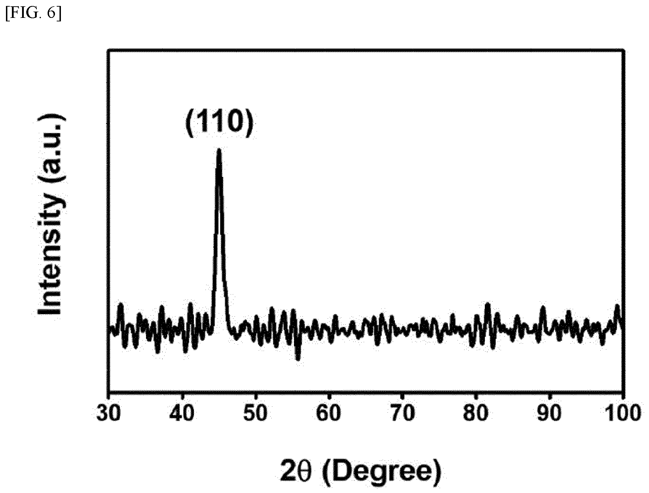

FIG. 6 is an X-ray diffraction analysis graph of the nanohelix according to the present invention, and FIG. 7 is an HRTEM image of the nanohelix according to the present invention.

Referring to FIGS. 6 and 7, it can be seen that the nanohelix has a (110) crystalline plane of a body-centered cubic structure, and has an average lattice interval of about 2.02 $\pm$ 0.02 Å. Further, it can be seen that in order to promote the coupling with the isotropic integrin ligand, the diameter of the nanowire forming the nanohelix is almost similar to about 10 nm that is an integrin molecule size.

**Experimental Example 2**

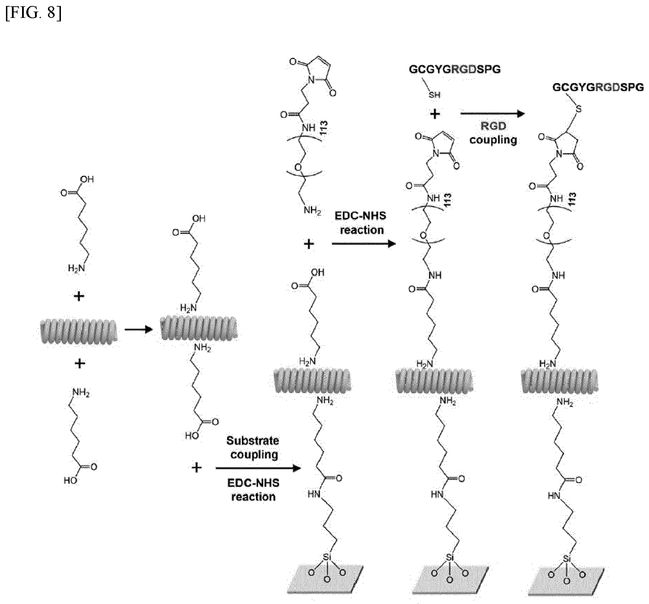

In order to confirm the characteristic of the nanohelix-substrate complex according to the present invention, the nanohelix-substrate complex was photographed with a Field Emission Scanning Electron Microscope (FE-SEM), the Fourier-Transform Infrared Spectroscopy (FT-IR) was carried, and the nanohelix-substrate complex was photographed with an Atomic Force Microscope (AFM), and the results thereof are represented in FIGS. 8 to 11.

The FT-IR was conducted by using GX1 (Perkin Elmer Spectrum, USA) in order to confirm the chemical bond characteristics of the nanohelices. The samples analyzed for the change in chemical bond characteristics were lyophilized and densely packed into KBr pellet prior to the analysis.

FIG. 8 is an image schematically illustrating an operation of preparing the nanohelix-substrate complex according to the present invention. Referring to FIG. 8, aminocaproic acid was coupled to the nanohelix. Next, the aminocaproic acid-bonded nanohelix was put in water containing EDC and NHS and activated by using the EDC/NHS reaction, and then was coupled to the substrate of which the surface is aminated. Polyethylene glycol was coupled to aminocaproic acid coupled to the nanohelix that is not coupled with the substrate, and the integrin ligand was coupled to the nanohelix by reacting the polyethylene glycol and the thiolated integrin ligand (RGD).

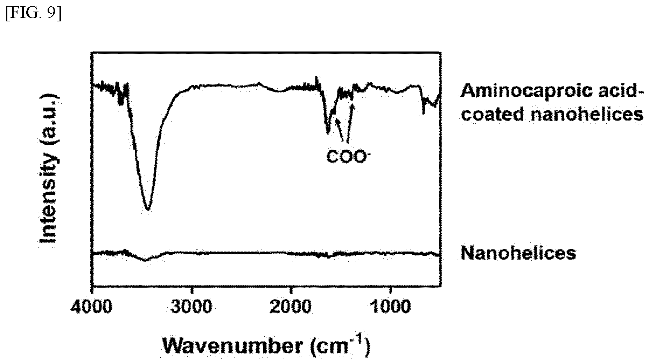

FIG. 9 is a diagram illustrating an FT-IR analysis result of the nanohelix-substrate complex according to the present invention. Referring to FIG. 9, the chemical bond characteristics of the aminocaproic acid-coated nanohelix can be recognized. In particular, COO$^-$ binding was confirmed at 1560 - 1565 cm$^{-1}$ and 1387 - 1389 cm$^{-1}$. Through this, it can be seen that aminocaproic acid was successfully coupled to the nanohelix.

In addition, in order to minimize adhesion of non-ligand-specific macrophages, the substrate that is not coupled with the nanohelix was coupled with a methoxy-PEG-NHS ester group to be inactivated, and referring to FIG. 3, the uniform distribution of the nanohelices can be confirmed through the scanning electron microscope, and it can be seen that a density of the nanohelices is about 62802 $\pm$ 2385 nanohelices/mm$^2$

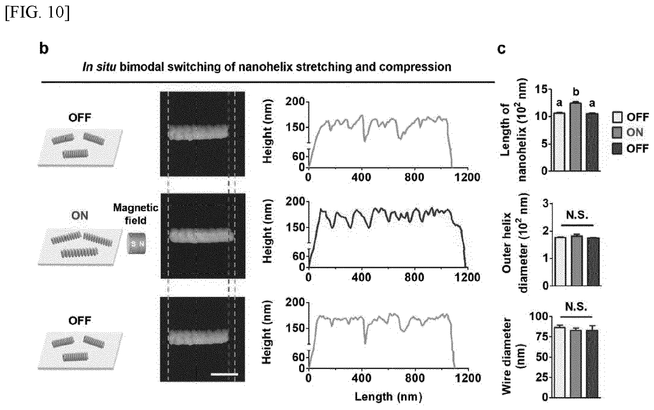

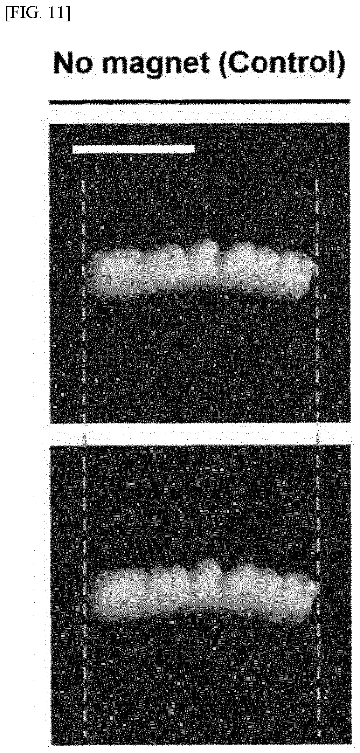

FIG. 10 is a diagram illustrating the result obtained by using the AFM in order to confirm magnetic bimodal switching of an elastic motion with stretching ("ON") and compression ("OFF") of the nanohelix according to the present invention. FIG. 11 is a diagram illustrating the result obtained by photographing the case where a magnetic field is not applied to the nanohelix according to the present invention by using the AFM.

Referring to FIGS. 10 and 11, it can be seen that in the nanohelix-substrate complex according to the present invention, when a magnetic field is applied, the nanohelix is stretched, so that the length of the nanohelix increases, and when the magnetic field is not applied again, the nanohelix is compressed, so that the length of the nanohelix returns to the original state. However, it can be seen that only the length of the nanohelix is simply increased and then decreased again, but the outer diameter of the nanohelix or the diameter of the nanowire forming the nanohelix is not significantly different.

In particular, it can be seen that the length of the nanohelix before the application of the magnetic field is $1060 \pm 9$ nm and the length of the nanohelix when the magnetic field is applied is $1243 \pm 25$ nm, and when the magnetic field is not applied again, the length of the nanohelix is decreased to $1052 \pm 9$ nm. In this case, the diameter of the nanohelix is maintained with 174 nm to 181 nm, and the diameter of the nanowire forming the nanohelix is maintained with 83 to 86 nm, so that it can be seen that the outer helix diameter and the wire diameter of the nanohelix remained similar without significant differences during the cyclic switching "OFF", "ON", and "OFF".

Through this, it can be seen that in the nanohelix-substrate complex of the present invention, the macroscale ligand density is constantly maintained during the bimodal switching.

**Experimental Example 3**

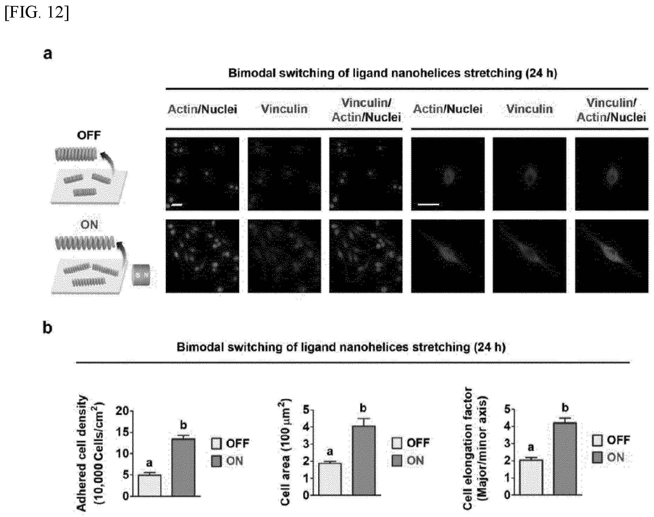

The following experiment was conducted to confirm an influence on the adhesion of macrophages according to the application of a magnetic field to the nanohelix-substrate complex according to the present invention, and the result thereof is represented in FIGS. 12 to 15.

The effect of controlling adhesion and phenotypic polarization of macrophages under the bimodal switching of the nanohelix was accessed. The substrate was subjected to sterilization under ultraviolet light for 2 hours prior to the culture. Macrophages (RAW 264.7, passage 5, ATCC) were seeded onto the sterilized substrate at the density of about $9 \times 10^4$ cells/cm$^2$, and were cultured under basal growth medium containing high glucose Dulbecco's Modified Eagle Medium (DMEM) with 10% heat-inactivated fetal bovine serum, and 50 IU/mL penicillin and streptomycin antibiotics at 37 °C under 5% $CO_2$. Bimodal switching-regulated adhesion of macrophages was accessed under cyclic switching between "ON" state (placing a permanent magnet (270 mT) near the edge of the substrates, in which the ligand-bearing nanohelices were stretched toward the edge of substrates) and "OFF" state (withdrawing the magnet from the substrate, in which the ligand-bearing nanohelices were compressed to revert to their original nanostructures). Control experiments to assess macrophage adhesion were conducted under bimodal switching, but were conducted in the absence of the nanohelices or integrin ligands.

M1 medium used for assessment of the phenotypic polarization of macrophages was prepared by using a basal medium with 10 ng/mL each of lipopolysaccharide (LPS) and recombinant interferon-gamma (IFN-$\gamma$). M2 medium was prepared by using a basal medium with 20 ng/mL each of interleukin-4 (IL4) and interleukin-13 (IL-13). The adhesion-assisted M2 phenotypic polarization of the macrophages was assessed with inhibitors of ROCK (50 $\mu$M Y27632), myosin II (10 $\mu$M blebbistatin), or actin polymerization (2 $\mu$g/mL of cytochalasin D).

FIG. 12 is a confocal immunofluorescent image (a) of F-actin, nuclei, and vinculin in macrophages cultured (for 24 hours) by using the nanohelix-substrate complex according to the present invention, and a graph (b) illustrating a cell density, a cell area, and a cell elongation factor calculated based on the result of the confocal immunofluorescent experiment, and a scale bar indicates 20 $\mu$m.

Referring to FIG. 12, in the confocal immunofluorescent image, the macrophages have considerably larger cell adhesion density and vinculin and F-actin spread areas in the "ON" mode in which a magnetic field is applied in the bimodal switching, compared to the "OFF" mode in which a magnetic field is not applied, which shows the adhesion of macrophages was promoted when the magnetic field is applied.

Through this, it was confirmed that the macroscale stretching-mediated macrophage adhesion may exhibit nanoscale stretching to promote the macrophage adhesion.

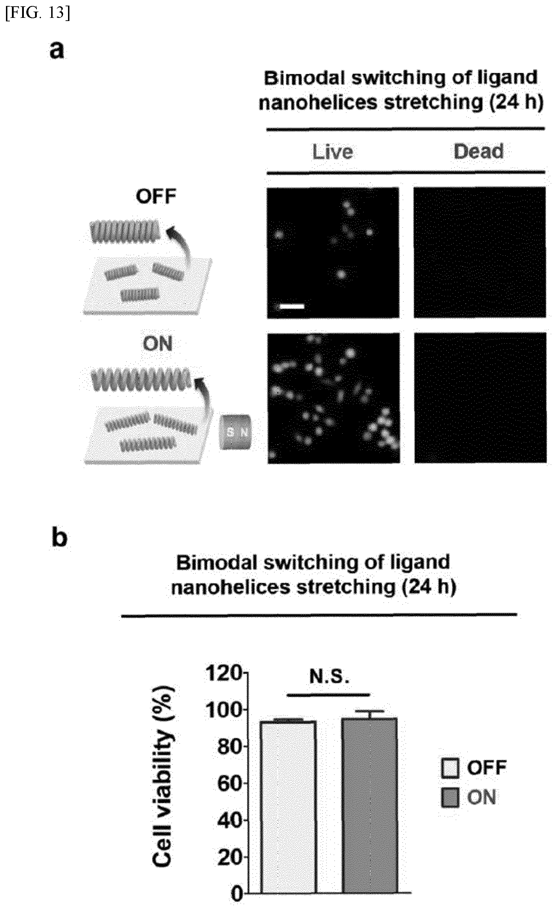

FIG. 13 is a confocal immunofluorescent image (a) for live cells and dead cells of macrophages cultured (for 24 hours) by using the nanohelix-substrate complex according to the present invention, and a graph (b) illustrating cell viability calculated based on the result of the confocal immunofluorescent experiment, and a scale bar represents 50 $\mu$m.

Referring to FIG. 13, it can be seen that even in the bimodal switching in which the magnetic field is applied, cell viability is excellent at 95%, so that the nanohelix-substrate complex has excellent cellular compatibility for macrophages.

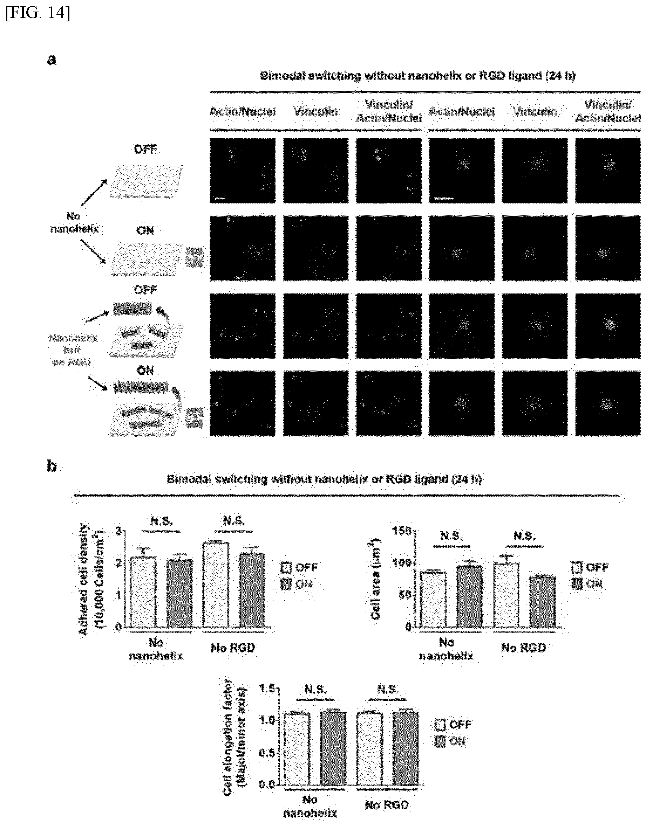

FIG. 14 is a diagram illustrating a result of an experiment for adhesion of macrophages for bimodal switching in a substrate having no nanohelix or the nanohelix-substrate complex to which the integrin ligand (RGD) is not coupled according to a comparative example of the present invention, and a of FIG. 14 is a confocal immunofluorescent image of F-actin, nuclei, and vinculin in macrophages cultured for 24 hours, and b of FIG. 14 is a graph representing a cell density, a cell area, and a cell elongation factor calculated based on the confocal immunofluorescent experiment result, and in this case, a scale bar represents 20 $\mu$m.

Referring to FIG. 14, in the comparative example, there is no significant difference in the bimodal switching "ON" and "OFF" in the state of using the substrate having no nanohelix or the substrate to which the integrin ligand (RGD) is not coupled, so that the adhesion of macrophages is not promoted.

Through this, in the case of the nanohelix-substrate complex of the present invention, it can be seen that the bimodal switching exhibits an effect only when the integrin ligand is coupled to the nanohelix.

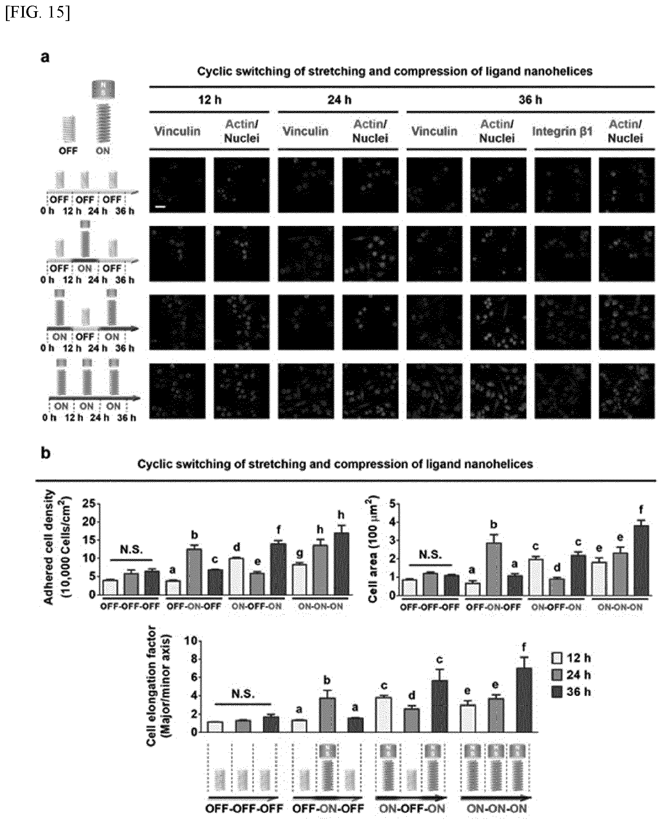

FIG. 15 is a confocal immunofluorescent image of F-actin, nuclei, and vinculin in macrophages cultured for 36 hours by regulating application of a magnetic field at an interval of 12 hours by using the nanohelix-substrate complex according to the present invention, and a scale bar represents 20 $\mu$m.

Referring to FIG. 15, it can be seen that in the case where a magnetic field is not applied, there is no change in cell adhesion, and in the case where a magnetic field is applied, cell adhesion is promoted, and in the case where a magnetic field is not applied again, cell adhesion is reversibly decreased.

Through this, the nanohelix-substrate complex of the present invention temporally and reversibly promotes and suppresses the adhesion of macrophages through the stretch and the compression of the nanohelix.

**Experimental Example 4**

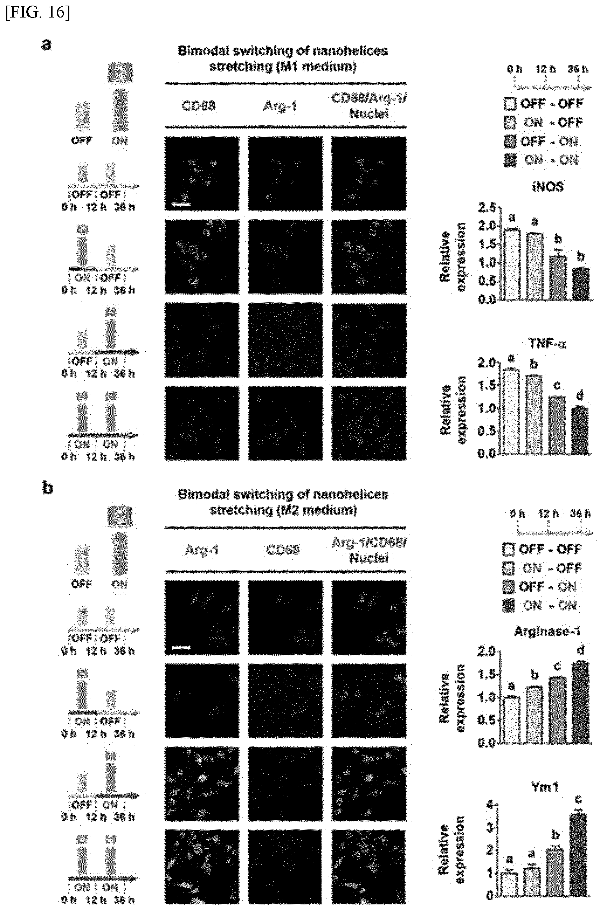

An experiment on the control of the nano-periodicity controls phenotypic polarization-mediated adhesion of macrophages by using the nanohelix-substrate complex according to the present invention was performed as described below, and the result thereof is represented in FIGS. 16 to 22.

The adhesive structures of macrophages are known to modulate their phenotypic polarization in the presence of M1 or M2 polarization stimulators. In particular, macrophages that develop robust adhesion structures, including the assembly of prevalent F-actin and vinculin in elongated shapes, are prone to activate their phenotypic polarization into regenerative/anti-inflammatory M2 state. In contrast, macrophages with low F-actin and rounded shape are prone to activate the M1 state.

FIG. 16 is a result of an experiment for control of macrophage adhesion-dependent phenotypic polarization of macrophages by application of a magnetic field by using the nanohelix-substrate complex according to the present invention.

Referring to FIG. 16, immunofluorescent confocal images revealed that macrophages gradually exhibited weaker CD68 fluorescence signals in M1-induction medium as a magnetic field is applied, but exhibited stronger Arg-1 fluorescence signals in M2-induction medium as a magnetic field is applied. Further, it can be seen that gene expression profiles were prone to be observed in the immunofluorescence. The macrophage exhibited the considerably low iNOS and TNF-$\alpha$ expression in the M1-induction medium as a magnetic field is applied, but exhibited high Arg-1 and Ym1 expression in the M2-induction medium as a magnetic field is applied.

Through this, it can be seen that the bimodal switching of the nanohelix controlling macrophage adhesion according to the application of the magnetic field may control polarization dependent on adhesion of macrophages.

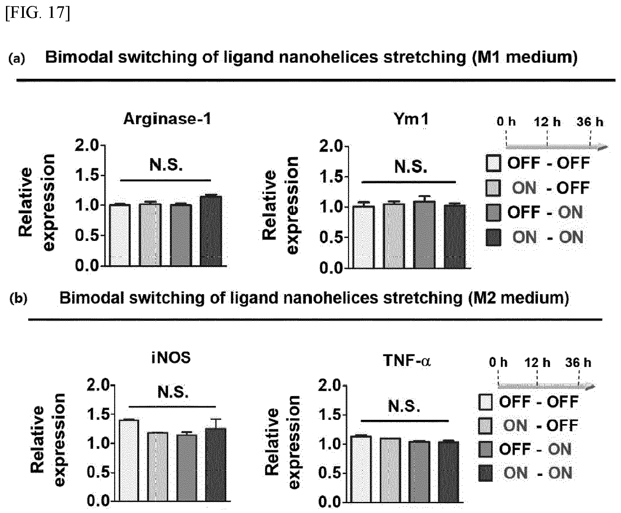

FIG. 17 is a result of an experiment for controlling application of a magnetic field when there is no stimulation medium matched to a polarization phenotype (that is, M1 expression in an M2-stimulation medium or M2 expression in an M1-stimulation medium) by using the nanohelix-substrate complex according to the present invention.

Referring to FIG. 17, it was found that the controlling of the application/non-application of the magnetic field under no presence of the stimulation medium matched with the polarization phenotype (that is, M1 expression in the M2-stimulation medium or M2 expression in the M1-stimulation medium) did not exhibit a meaningful difference in iNOS, TNF-$\alpha$, Arg-1, and Ym1 expression.

Through this, it can be seen that M1 and M2 stimulation does not affect the change in M1 or M2 expression without appropriate stimulation, respectively.

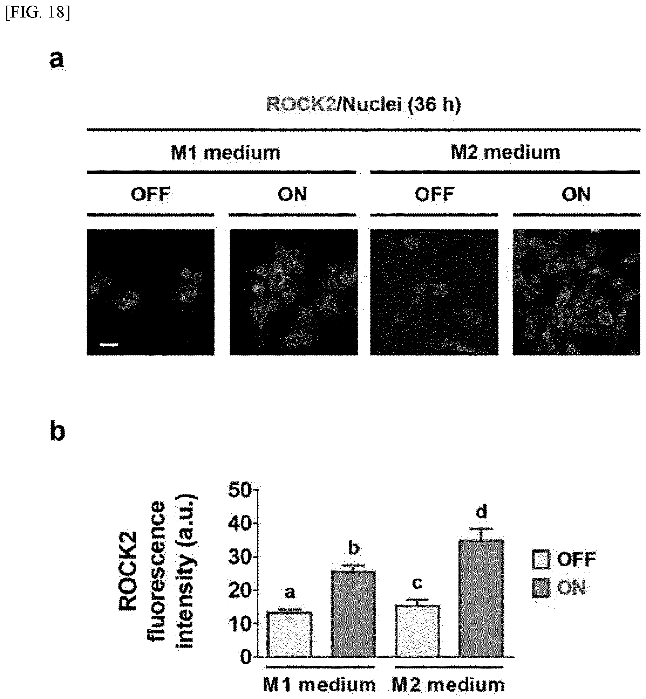

FIG. 18 is a confocal immunofluorescent image (a) of ROCK2 and nuclei after macrophages are cultured in an M1 or M2 medium under bimodal switching of stretching ("ON) in which a magnetic field is applied and compression ("OFF") in which a magnetic field is not applied for 36 hours, and a graph (b) illustrating ROCK2 immunofluorescent intensity calculated based on a result of the confocal immunofluorescent experiment, and a scale bar represents 20 $\mu$m.

Referring to FIG. 18, it can be seen that the application of the magnetic field elevated ROCK2 expression under M2 medium culture compared to the non-application of the magnetic field. Through this, the adhesion-dependent M2 polarization of macrophages is mediated by the stretching ("ON") condition in which the magnetic field is applied.

a of FIG. 19 is a confocal immunofluorescent image of CD68, F-actin, and nuclei in macrophages cultured in an M1 polarization medium with and without an inhibitor for ROCK (Y27632), myosin II (blebbistatin), or actin polymerization (cytochalasin D) for 36 hours against and a graph illustrating a cell area, a cell elongation factor, and CD68 fluorescence intensity calculated based on a confocal immunofluorescent experiment result, and b of FIG. 19 is a confocal immunofluorescent image of Arg-1, and F-actin, and nuclei in macrophages cultured in an M2 polarization medium with and without an inhibitor for ROCK (Y27632), myosin II (blebbistatin), or actin polymerization (cytochalasin D) and a graph illustrating a cell area, a cell elongation factor, and Arg-1 fluorescence intensity calculated based on the confocal immunofluorescent experiment result, and a scale bar represents 20 $\mu$m.

Referring to FIG. 19, it can be seen that under the M1 medium culture using pharmacological inhibitors (Y27632, blebbistatin, or cytochalasin D) that inhibit ROCK, myosin II, or actin polymerization, the M1 polarization suppression of macrophages is continuously hindered under the bimodal switching "ON" state. Further, it can be seen that under the M2 medium culture, when the inhibitor is treated, stimulation of robust adhesion (higher cell area and elongation factor) and expression of M2 polarization (Arg-1 expression) are hindered.

Through this, ROCK, myosin II, and F-actin function as molecular switches in the regulation of bimodal M1 vs. M2 polarization in concert with the magnetic bimodal switching of the integrin ligand-bearing nanohelices.

## Experimental Example 5

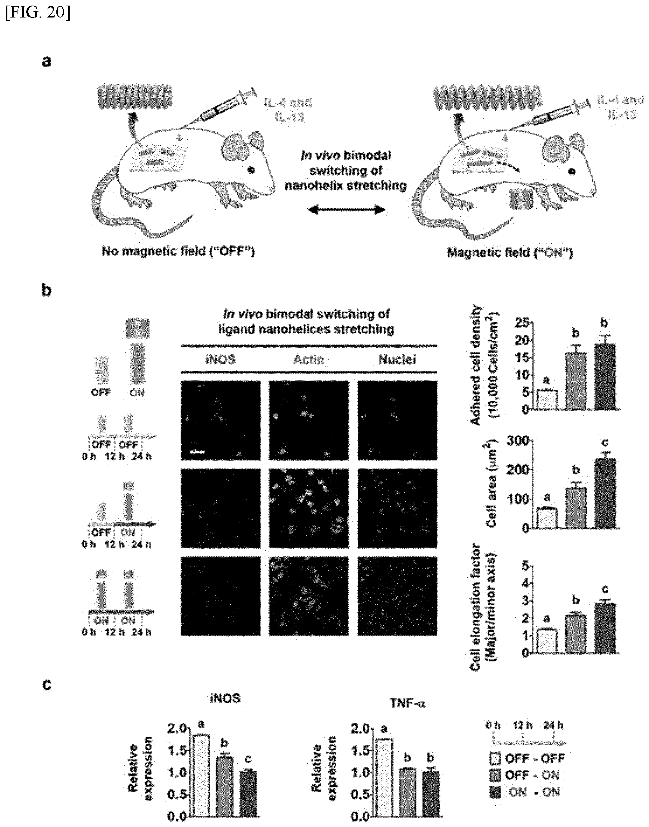

A following experiment was conducted in order to confirm the spatial control of adhesion and phenotype of host macrophages in vivo through stretching and compression of the nanohelix according to the application of a magnetic field by using the nanohelix-presenting substrate according to the present invention, and a result thereof is presented in FIGS. 20 to 22.

FIG. 20 is a diagram illustrating a result of an experiment for control of adhesion and phenotype of host macrophages in vivo by using the nanohelix-substrate complex according to the present invention. a of FIG. 20 is a schematic diagram illustrating the case where a magnetic field is applied to the nanohelix-substrate complex in vivo, and in this case, both interleukin-4 and interleukin-13 (M2 inducing agent) were injected onto the subcutaneous implanted substrate in vivo. b of FIG. 20 is a confocal immunofluorescent image of iNOS, F-actin, and nuclei in host macrophage adhered to the substrate after 24 hours under the bimodal switching of stretching ("ON") and compression ("OFF") of the nanohelix and a quantitative graph thereof, and a scale bar represents 20 $\mu$m. c of FIG. 20 is a graph of an quantitative analysis result of the adherent host macrophage in vivo of M1 phenotypic marker (iNOS and TNF-$\alpha$) adhered to the substrate after 24 hours under the bimodal switching of stretching ("ON") and compression ("OFF") of the nanohelix.

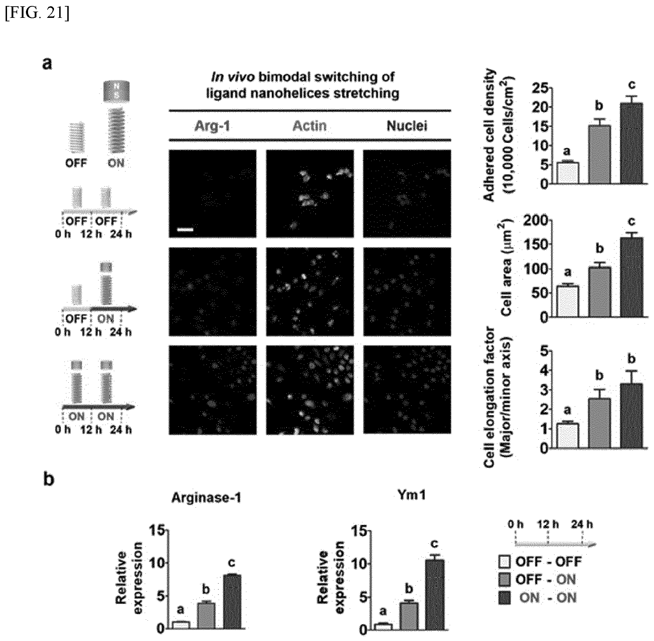

a of FIG. 21 is a confocal immunofluorescent image of Arg-a, F-actin, and nuclei in host macrophage adhered to the substrate after 24 hours under the bimodal switching of stretching ("ON") and compression ("OFF") of the nanohelix, and a scale bar represents 20 $\mu$m. b of FIG. 21 is a graph of an quantitative analysis result of the adherent host macrophage in vivo of M2 phenotypic marker (Arg-1 and Ym1) adhered to the substrate after 24 hours under the bimodal switching of stretching ("ON") and compression ("OFF") of the nanohelix.

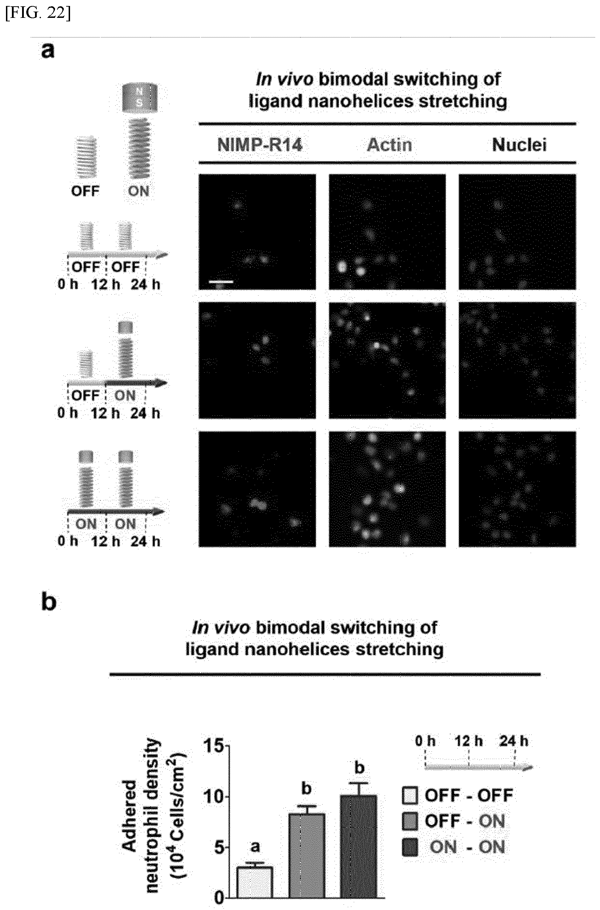

FIG. 22 is a diagram illustrating a result of an experiment of adhesion in vivo of host neutrophil to the substrate by using the nanohelix-substrate complex according to the present invention. a of FIG. 22 is an immunofluorescent confocal image of NIMP-R14, F-actin, and the nuclei in host macrophages adhered to the substrate after 24 hours, and in this case, a scale bar represents 20 $\mu$m. b of FIG. 22 is a graph of quantification data of in vivo adherent NIMP-R14-positive host neutrophils, and both interleukin-4 and interleukin-13 were injected onto the subcutaneously implanted substrate coupled with the nanohelix.

Referring to FIGS. 20 to 22, it can be seen that the stretching ("ON") in which a magnetic field is applied in the bimodal switching exhibits significantly higher adherent cell density, F-actin spread area, more pronounced elongation, and low iNOS and TNF-$\alpha$ expression, compared to the compression "OFF") in which a magnetic field is not applied to promote time-regulated adhesion that inhibits M1 polarization of host macrophages. Further, it can be seen that the stretching ("ON") in which a magnetic field is applied in the bimodal switching has a robust adherent structure, and temporarily stimulates adhesion-mediated M2 polarization of host macrophages exhibiting significantly high Arg-1 and Ym1 expression associated with adherent NIMP-R14 positive neutrophils, compared to the compression ("OFF") in which a magnetic field is not applied.

## Experimental Example 6

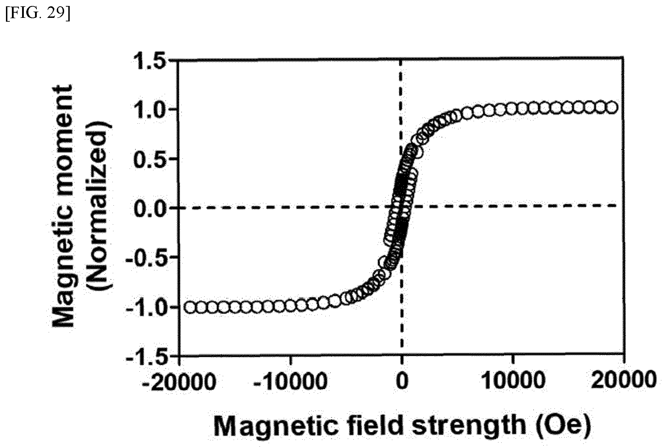

In order to check the form and the chemical characteristic of the nanohelix according to the present invention, the prepared nanohelix was photographed by using a Scanning Electron Microscope (SEM), a High-Angle Annular Dark-Field Scanning Transmission Electron Microscopy (HAADF-STEM), a High-Resolution Transmission Electron Microscope (HR-TEM), and a High-Resolution Scanning Transmission Electron Microscopy (HR-STEM), and then analyzed by using Energy Dispersive X-ray Spectroscopy (EDS), Electron Energy Loss Spectroscopy (EELS), Vibrating-Sample Magnetometry (VSM), and X-ray Diffraction (XRD), and a result thereof is represented in FIGS. 22 and 29.

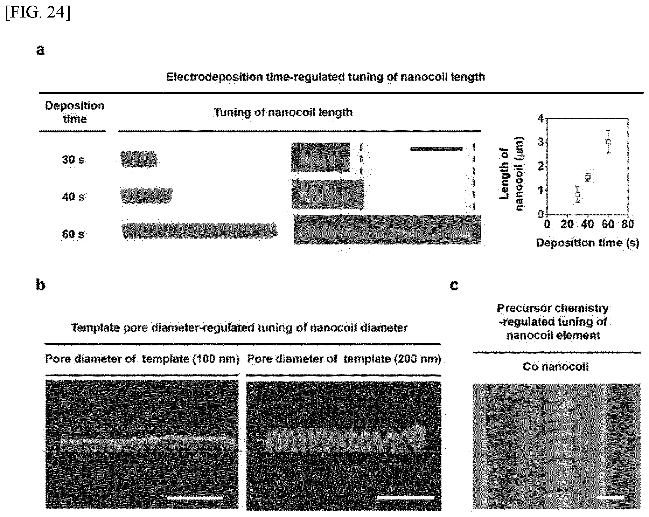

FIG. 24 is an SEM image of the nanohelix according to the present invention. In particular, a of FIG. 24 is an SEM image and a graph of the measured length of the CoFe nanohelix according to an electrodeposition time, b of FIG. 24 is an SEM image of the CoFe nanohelix prepared by adjusting a pore diameter of the electrodeposition template, and c of FIG. 2 is an SEM image of the cobalt nanohelix and the CoFe nanohelix, and in this case, a scale bar represents 1 $\mu$m in a of FIG. 24, 500 nm in b of FIG. 2, and 200 nm in c of FIG. 2.

Referring to FIG. 24, according to the nanohelix according to the present invention, it can be seen that it is possible to regulate the diameter of the CoFe nanohelix according to the pore diameter of the electrodeposition template, it is possible to control a constituent element of the nanohelix according to the control of the metal ion precursor, and it is possible to regulate the length of the CoFe nanohelix according to an electrodeposition time..

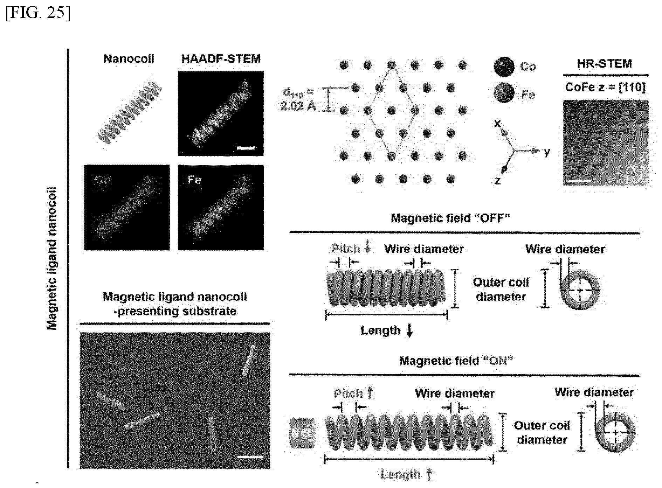

FIG. 25 is an HAADF-STEM image, an EDS mapping image, and an HR-STEM image of the nanohelix according to the present invention.

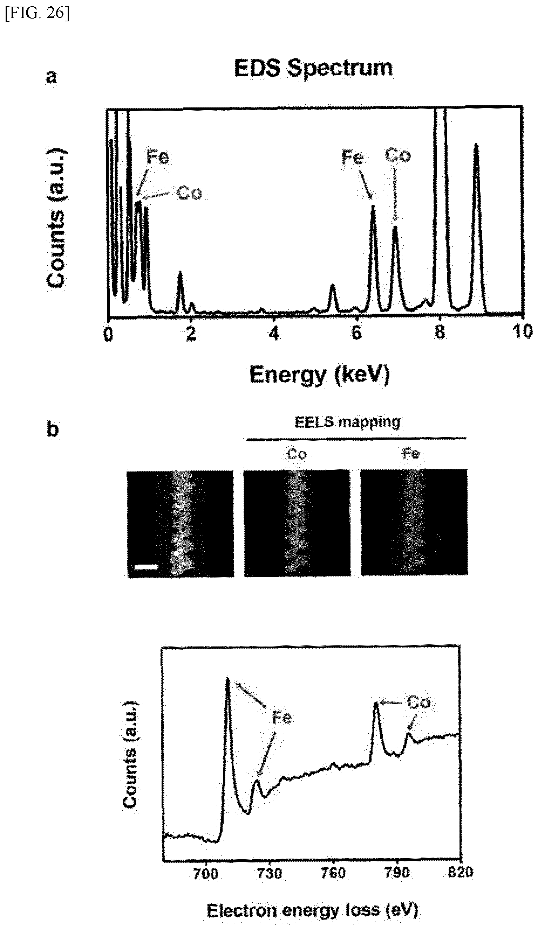

FIG. 26 is a graph (a) illustrating an EDS analysis result, and a graph and a mapping image (b) illustrating an EELS analysis result of the nanohelix according to the present invention.

Referring to FIGS. 25 and 26, in the HAADF-STEM and EELS maaping image, it can be seen that the nanohelix consists of cobalt (Co) and iron (Fe), each of which is constantly distributed with a distribution of about 50 atom%.

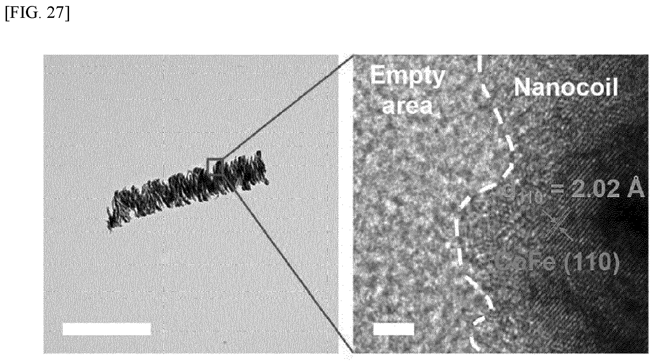

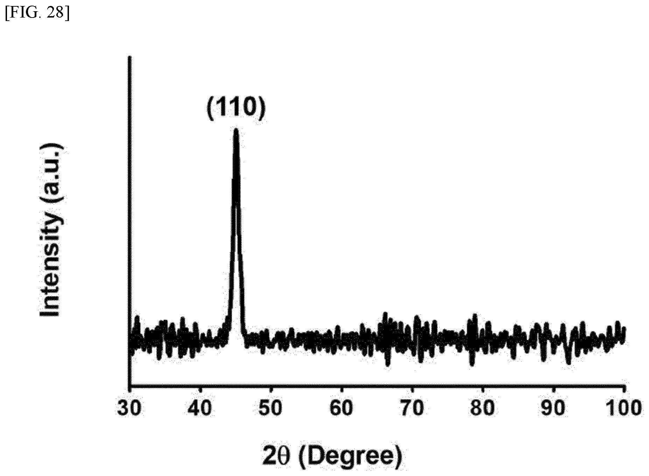

FIG. 27 is a High-Resolution Transmission Electron Microscopy (HRTEM) image of the nanohelix according to the present invention, and FIG. 28 is an X-ray diffraction analysis graph of the nanohelix according to the present invention.

Referring to FIGS. 27 and 28, it can be seen that the nanohelix has a (110) crystal plane of a body centered cubit structure, and has an average lattice interval of about 2.02 ± 0.02 Å. Further, it can be seen that in order to promote the coupling with the isotropic integrin ligand, the diameter of the nanowire forming the nanohelix is almost similar to about 10 nm that is an integrin molecule size.

FIG. 29 is a graph illustrating a vibrating-sample magnetometry measurement result of the nanohelix according to the present invention. In particular, the magnetic characteristic of the nanohelix by cobalt and iron was confirmed, and through this, it can be recognized that reversible bimodal switching between nano stretching ("ON") and nano-compression ("OFF) of the nanohelix is possible.

**Experimental Example 7**

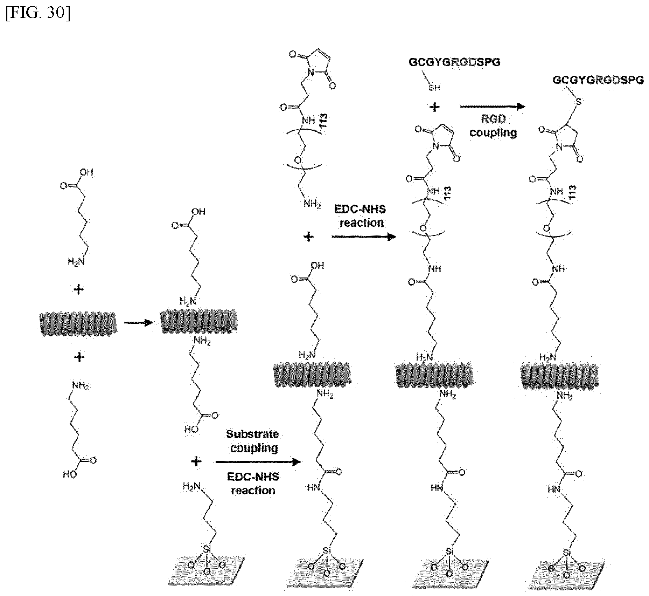

In order to confirm the characteristic of the nanohelix-substrate complex according to the present invention, the nanohelix-substrate complex was photographed with a Field Emission Scanning Electron Microscope (FE-SEM), the Fourier-Transform Infrared Spectroscopy (FT-IR) was carried, and the nanohelix-substrate complex was photographed with an Atomic Force Microscope (AFM), and the results thereof are represented in FIGS. 30 to 33.

The FT-IR was conducted by using GX1 (Perkin Elmer Spectrum, USA) in order to confirm the chemical bond characteristics of the nanohelices. The samples analyzed for the change in chemical bond characteristics were lyophilized and densely packed into KBr pellet prior to the analysis.

FIG. 30 is an image schematically illustrating an operation of preparing a nanohelix-substrate complex according to the present invention. Referring to FIG. 30, aminocaproic acid was bonded to the nanohelix. Next, the aminocaproic acid-bonded nanohelix was put in water containing EDC and NHS and activated by using the EDC/NHS reaction, and then was coupled to the substrate of which the surface is aminated. Polyethylene glycol was coupled to aminocaproic acid coupled to the nanohelix that is not coupled with the substrate, and the integrin ligand was coupled to the nanohelix by reacting the polyethylene glycol and the thiolated integrin ligand (RGD).

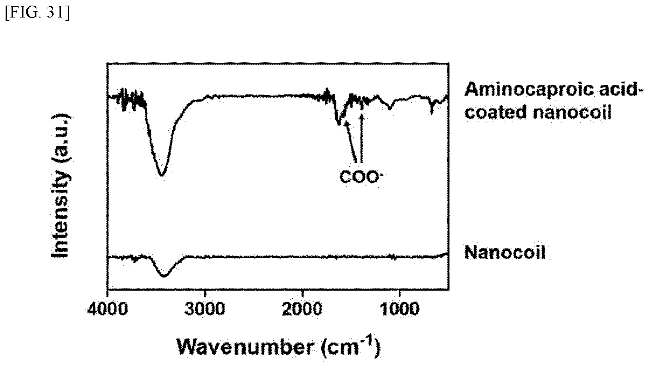

FIG. 31 is a diagram illustrating an FT-IR analysis result of the nanohelix-substrate complex according to the present invention. Referring to FIG. 31, the chemical bond characteristics of the aminocaproic acid-coated nanohelix can be recognized. In particular, $COO^-$ binding was confirmed at 1560 - 1565 $cm^{-1}$ and 1387 - 1389 $cm^{-1}$. Through this, it can be seen that aminocaproic acid was successfully coupled to the nanohelix.

In addition, in order to minimize adhesion of non-ligand-specific stem cells, the substrate that is not coupled with the nanohelix was coupled with a methoxy-PEG-NHS ester group to be inactivated, and referring to FIG. 25, the uniform distribution of the nanohelices can be confirmed through the scanning electron microscope, and it can be seen that a density of the nanohelices is about 62802 ± 2385 nanohelices/$mm^2$

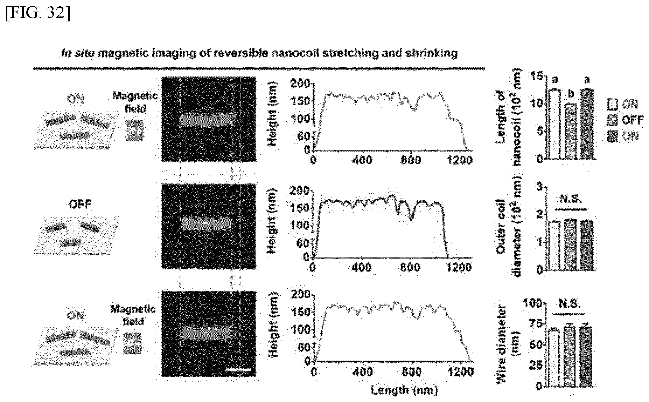

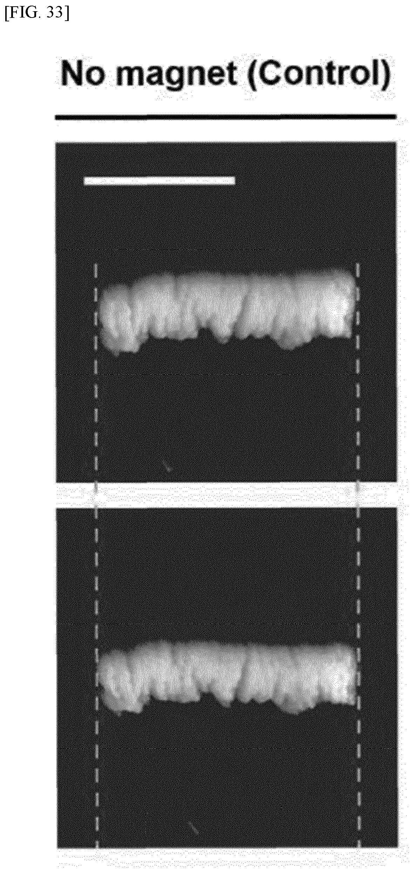

FIG. 32 is a diagram illustrating the result obtained by using the AFM in order to confirm magnetic bimodal switching of an elastic motion with stretching ("ON") and compression ("OFF") of the nanohelix according to the present invention. FIG. 33 is a diagram illustrating the result obtained by photographing the case where a magnetic field is not applied to the nanohelix according to the present invention by using the AFM.

Referring to FIGS. 32 and 33, it can be seen that in the nanohelix-substrate complex according to the present invention, when a magnetic field is applied, the nanohelix is stretched, so that the length of the nanohelix increases, and when the magnetic field is not applied again, the nanohelix is compressed, so that the length of the nanohelix returns to the original state. However, it can be seen that only the length of the nanohelix is simply increased and then decreased again, but the outer diameter of the nanohelix or the diameter of the nanowire forming the nanohelix is not significantly different.

In particular, it can be seen that the length of the nanohelix when the magnetic field is applied is 1243 ± 28 nm, when the magnetic field is not applied, the length of the nanohelix is decreased to 995 ± 4 nm, and when the magnetic field is applied again, the length of the nanohelix is increased to 1255 ± 18nm. In this case, the diameter of the nanohelix is maintained with 174 nm to 180 nm, and the diameter of the nanowire forming the nanohelix is maintained with 66 to 71 nm, so that it can be seen that the outer diameter and the wire diameter of the nanohelix remained similar without significant differences during the cyclic switching "OFF", "ON", and "OFF".

Through this, it can be seen that in the nanohelix-substrate complex of the present invention, the macroscale ligand density is constantly maintained during the bimodal switching.

**Experimental Example 8**

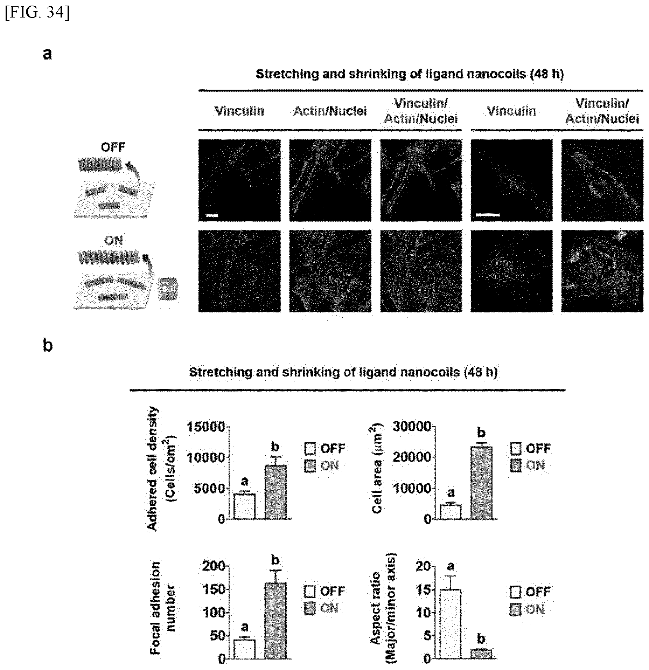

The following experiment was conducted to confirm an influence on the adhesion of stem cells according to the application of a magnetic field to the nanohelix-substrate complex according to the present invention, and the result thereof is represented in FIGS. 34 to 38.

To investigate the influence of magnetic switching of reversible strength and compression of the ligand-containing nanohelix on focal adhesion, mechanosensing and differentiation of the stem cells, the evaluation was conducted by using the nanohelix-substrate complex prepared in the preparation example. The substrate was sterilized under ultraviolet light for 2 hours prior to the use of the substrate. Human mesenchymal stem cells (hMSCs, passage 5 from Lonza) were plated on the sterilized substrate at a density of approximately 9,500 cells/cm$^2$ and cultured in growth medium containing high glucose Dulbecco's Modified Eagle Medium (DMEM) supplemented with 10% fetal bovine serum, 4 mM L-glutamine, and 50 U/mL penicillin/streptomycin at 37°C under 5% $CO_2$.

The focal adhesion and mechanosensing of the stem cells was investigated by placing a permanent magnet (270 mT) near the edge of the materials ("ON") for 48 hours to promote the in situ stretching of the nanohelices toward the edge of the materials or removing the magnet ("OFF") for 48 hours to induce reversible compression of the nanohelices to the original structures. The control experiment to evaluate the focal adhesion and mechanosensing of the stem cells was performed under bimodal switching (application/removal of the magnetic field), but was performed in the state where there was no nanohelix or integrin ligand.

FIG. 34 is a confocal immunofluorescent image (a) of F-actin, nuclei, and vinculin in stem cells cultured (after 48 hours) by using the nanohelix-substrate complex according to the present invention, and a graph (b) illustrating an adherent cell density, a cell area, focus adherence number, and an aspect ratio (a ratio of major axis/minor axis) calculated based on the result of the confocal immunofluorescent experiment, and a scale bar represents 50 $\mu$m.

Referring to FIG. 34, it can be seen that the stretching ("ON") mode in which the magnetic field is applied exhibits considerably higher adhesive cell density and focal adhesion throughout the wider area than the compression ("OFF") mode in which the magnetic field is removed, and promotes vinculin clustering in the focal adhesion complex.

The effect of cyclic switching "ON" (stretching) and "OFF" (compression) on the focal adhesion of stem cells was investigated under the placement ("ON") or removal ("OFF") of the magnet for 54 hours or the cyclic switching every 18 hours ("OFF-OFF-OFF", "OFF-ON-OFF", "ON-OFF-ON", and "ON-ON-ON" groups).

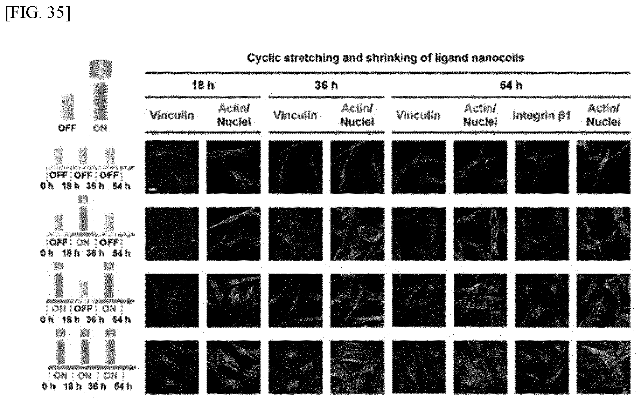

FIG. 35 is a confocal immunofluorescent image of F-actin, nuclei, and vinculin in stem cells cultured (after 54 hours) by changing the application of a magnetic field every 18 hours by using the nanohelix-substrate complex according to the present invention, and a scale bar represents 50 $\mu$m.

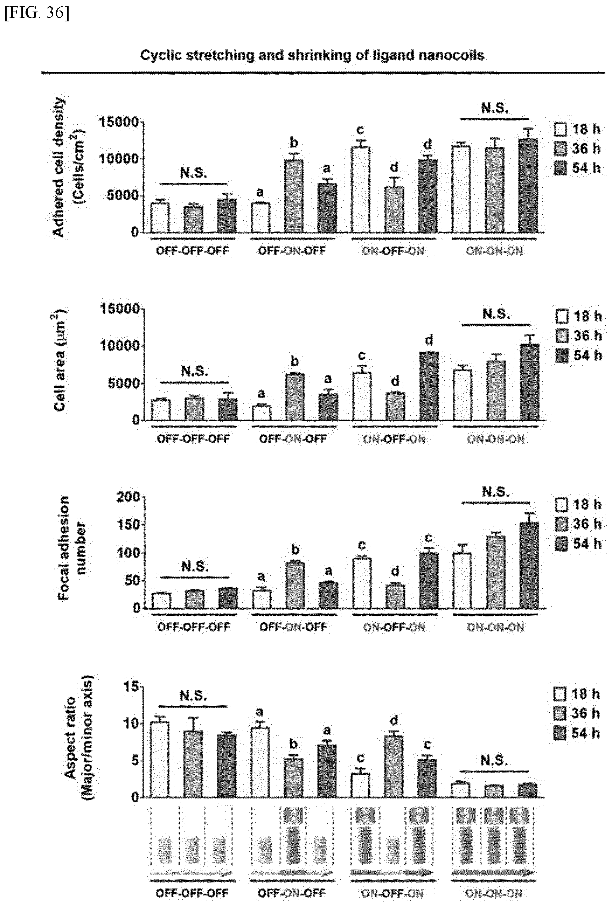

FIG. 36 is a graph illustrating an adherent cell density, a cell area, focal adherence number, and an aspect ratio (a ratio of major axis/minor axis) of the stem cells cultured by changing the application of a magnetic field every 18 hours by using the nanohelix-substrate complex according to the present invention calculated based on the result of the confocal immunofluorescent experiment.

Referring to FIGS. 35 and 36, "ON" (stretching) and "OFF" (compression) that are the bimodal switching of the nanohelix-substrate complex promote and suppress reversible integrin β1 expression and focal adhesion of stem cells in the repeated cycle, respectively. In particular, it can be seen that in the stretching mode in which the magnetic field is applied, the focal adhesion of the stem cells is promoted, and in the shrinking mode in which the magnetic field is removed, the focal adhesion of the stem cells is suppressed. Accordingly, it can be seen that in the case where the magnetic field is applied and then removed, adherent cell density, cell area, and focal adhesion number are increased and then decreased.

To confirm the cytocompatibility of the stretching and compression of CoFe nanohelices on adherent stem cells, cell staining was performed. The adherent stem cells on the ligand-coated CoFe nanohelices cultured under stretching ("ON") or compression ("OFF") conditions at 48 hour were washed with phosphate-buffered saline (PBS) and incubated in a staining solution containing 0.05% green fluorescent calcein-AM and 0.2% red-fluorescent propidium iodide (PI) in DMEM at 37°C for 30 minutes. The stained cells were washed with PBS and imaged under a fluorescence microscope. The live (green) and dead (red) cells were counted to determine viability of the stem cells.

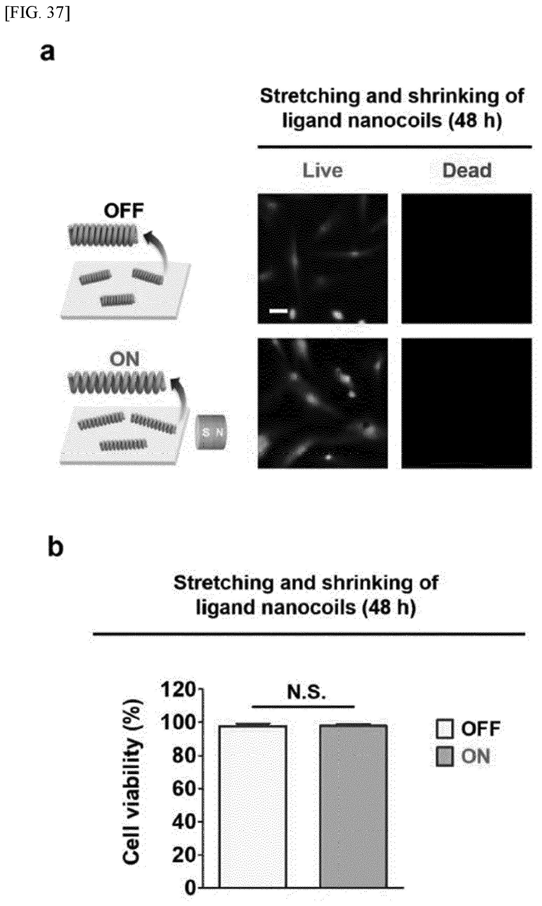

FIG. 37 is a confocal immunofluorescent image (a) of live cells and dead cells in stem cells cultured (after 48 hours) by using the nanohelix-substrate complex according to the present invention, and a graph (b) illustrating cell viability calculated based on the result of the confocal immunofluorescent experiment, and a scale bar represents 50 $\mu$m.

Referring to FIG. 37, it can be seen that cell viability is excellent at 95% in both the stretching mode in which the magnetic field is applied and the compression mode in which the magnetic field is not applied the CoFe nanohelix-substrate complex, so that the CoFe nanohelix-substrate complex does not have no cytotoxicity to stem cells, so that the cytocompatibility is excellent.

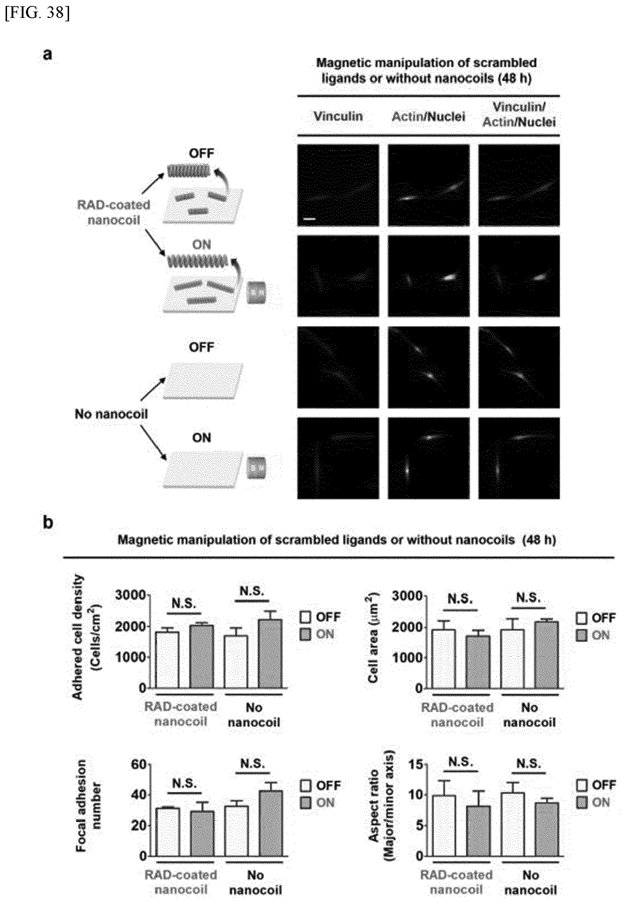

FIG. 38 is a diagram illustrating a result of an experiment for adhesion of stem cells for bimodal switching in a substrate having no nanohelix or the nanohelix-substrate complex to which the integrin ligand (RGD) is not coupled according to a comparative example of the present invention, and a of FIG. 38 is a confocal immunofluorescent image of F-actin, nuclei, and vinculin in stem cells cultured for 24 hours, and b of FIG. 38 is a graph representing an adherent cell density, a cell area, and focal adherence number, and an aspect ratio (a ratio of major axis/minor axis) calculated based on the confocal immunofluorescent experiment result, and in this case, a scale bar represents 50 $\mu$m.

Referring to FIG. 38, in the comparative example, there is no significant difference in the bimodal switching "ON" and "OFF" in the state of using the substrate having no nanohelix or the substrate to which the integrin ligand (RGD) is not coupled, so that the adhesion of stem cells is not promoted.

Through this, in the case of the nanohelix-substrate complex of the present invention, the bimodal switching exhibits an effect only when the integrin-coupled ligand peptide is coupled to the nanohelix, so that it can be seen that in order to promote and remotely control the stem cell adhesion, both the integrin-coupled ligand peptide and the nanohelix are required.

**Experimental Example 9**

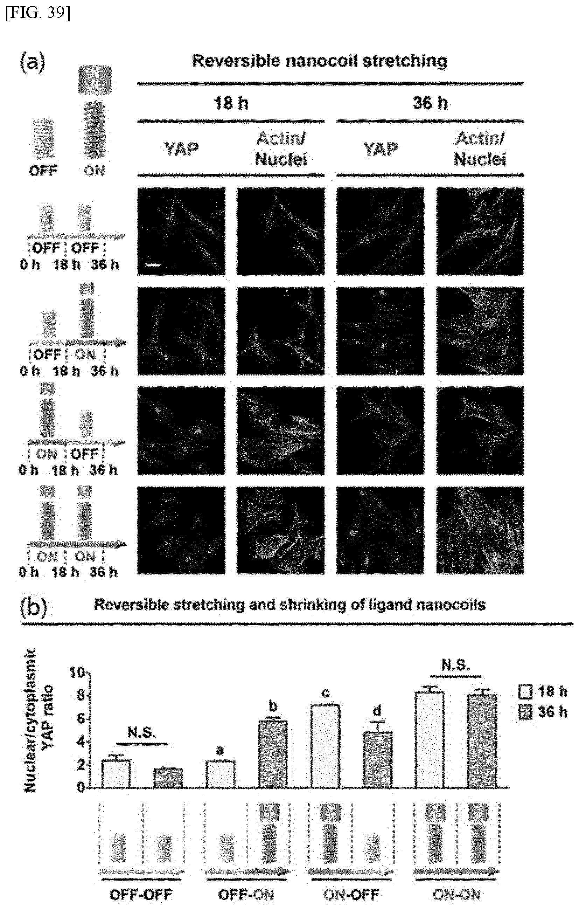

An experiment to find the effect of "ON" (stretching) and "OFF" (compression) that are the remote and reversible bimodal switching on osteogenic differentiation of stem cells by using the nanohelix-substrate complex according to the present invention was conducted as described below, and a result of the experiment is represented in FIGS. 39 to 46.

The integrin ligation-mediated focal adhesion and spreading of stem cells activate mechanotransduction signaling that mediates stem cell differentiation. Cyclic macroscale stretching of cell-adhesive fibronetin and laminin activates the phosphorylation of focal adhesion kinase (FAK) in stem cells to promote their osteogenic differentiation.

FIG. 39 is a confocal immunofluorescent image (a) of F-actin, nuclei, and vinculin in stem cells cultured for 36 hours by adjusting an application of a magnetic field at an interval of 18 hours by using the nanohelix-substrate complex according to the present invention, and a graph (b) illustrating nuclear/cytoplasmic YAP ratio calculated based on the result of the confocal immunofluorescent experiment, and a scale bar represents 50 $\mu$m.

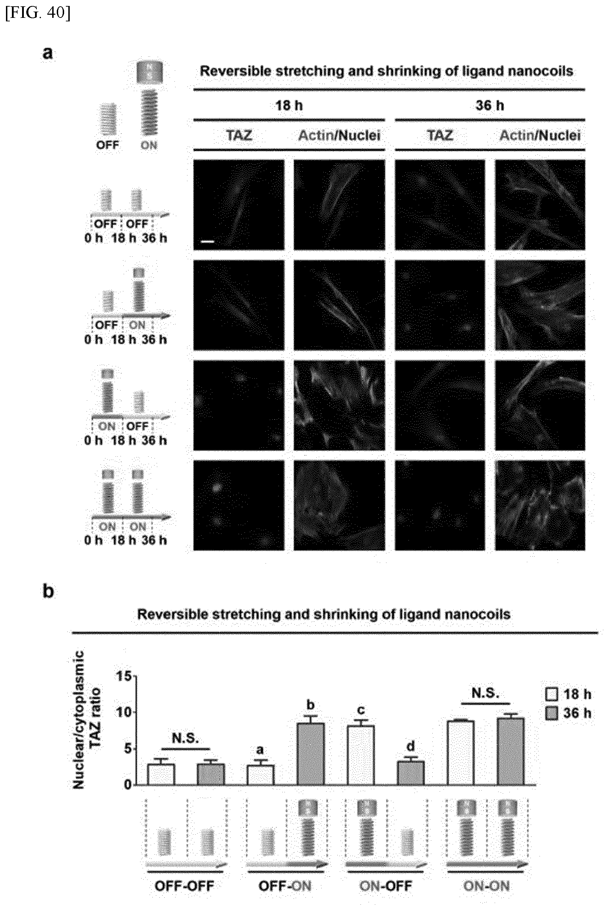

FIG. 40 is a confocal immunofluorescent image (a) of F-actin, nuclei, and TAZ in stem cells cultured for 36 hours by adjusting an application of a magnetic field at an interval of 18 hours by using the nanohelix-substrate complex according to the present invention, and a graph (b) illustrating nuclei/cytoplasm YAP ratio calculated based on the result of the confocal immunofluorescent experiment, and in this case, a scale bar represents 50 $\mu$m.

Referring to FIGS. 39 and 40, it can be confirmed that in the time-regulated bimodal switching, the stretching "ON" mode in which the magnetic field is applied stimulates significantly higher nuclear translocation of YAP/TAZ mechanotransducers of stem cells *via* immunofluorescence in a reversible manner.

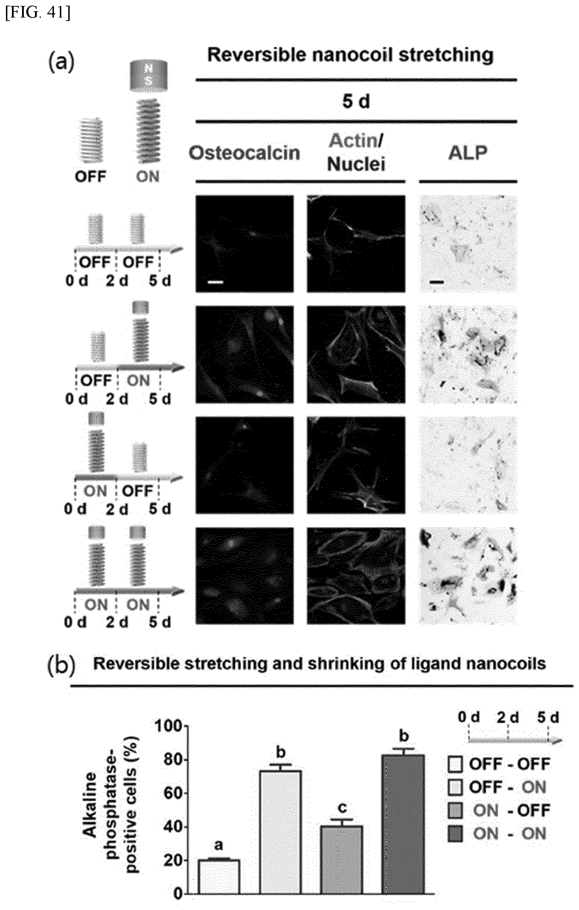

FIG. 41 is a confocal immunofluorescent image and ALP staining image (a) of osteocalcin, F-actin, nuclei in stem cells cultured 5 days by using the nanohelix-substrate complex according to the present invention, in which an application of a magnetic field is adjusted at the second day, and a graph (b) representing alkaline phosphatase-positive cell ratio calculated based on the result of the confocal immunofluorescent experiment, and in this case, a scale bar represents 50 $\mu$m.

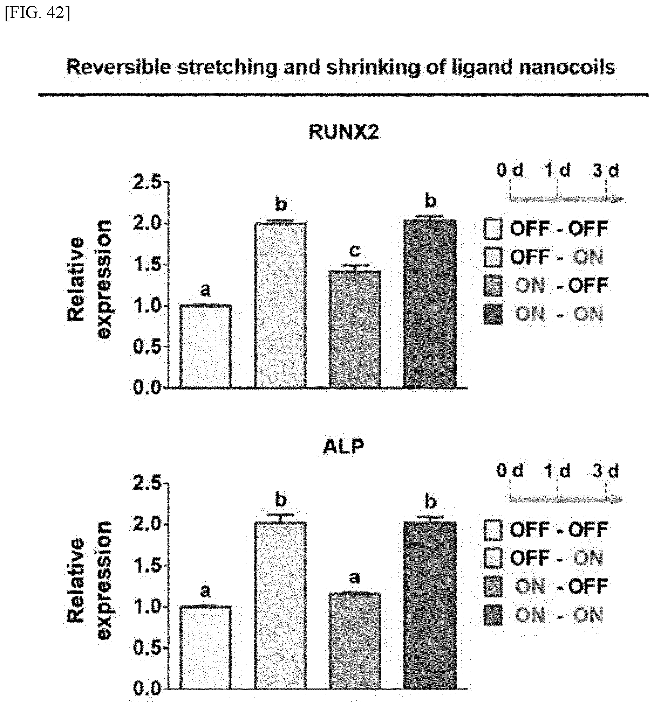

FIG. 42 is a graph illustrating a quantitative analysis of the nuclear/cytoplasmic RUNX2 and ALP gene expression profile in stem cells cultured for 3 days by using the nanohelix-substrate complex according to the present invention, in which an application of a magnetic field is adjusted after one day.

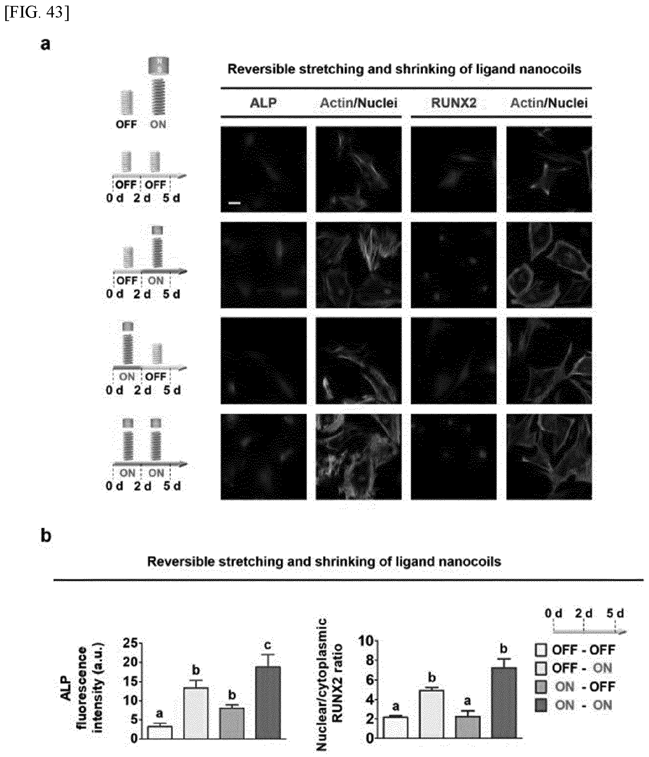

FIG. 43 is a confocal immunofluorescent image (a) of ALP genes, RUNX2, F-actin, and nuclei in stem cells cultured 5 days by using the nanohelix-substrate complex according to the present invention, in which an application of a magnetic field is adjusted at the second day, and a graph (b) representing ALP fluorescent intensity and nuclear/cytoplasmic YAP ratio calculated based on the result of the confocal immunofluorescent experiment, and in this case, a scale bar represents 50 $\mu$m.

Referring to FIGS. 42 and 43, in the time regulated bimodal switching, the stretching ("ON") in which the magnetic field is applied reversibly facilitates pronounced expression of early markers (significantly higher nuclear translocation in RUNX2, alkaline phosphatase-positive cells, and RUNX2/ALP gene expression) and late marker (pronounced osteocalcin expression) for osteogenic differentiation in stem cells.

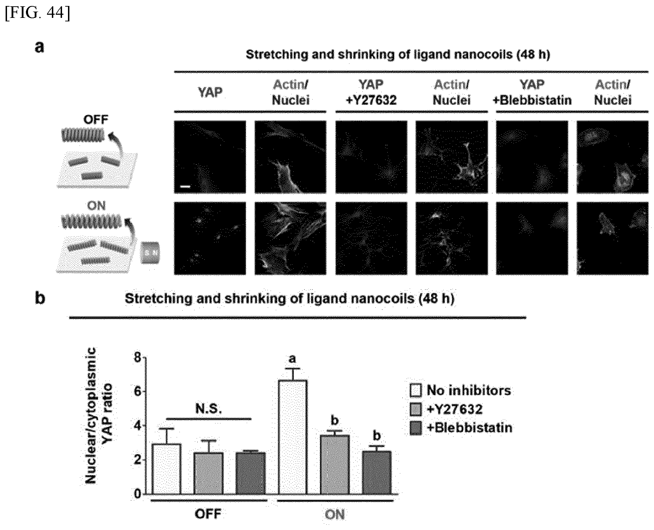

FIG. 44 is a confocal immunofluorescent image (a) of YAP, F-actin, and nuclei in stem cells cultured for 48 hours in a medium without an inhibitor and a medium with ROCK inhibitor (Y27632) and myosin II inhibitor (blebbistatin) by using the nanohelix-substrate complex according to the present invention, and a scale bar represents 50 $\mu$m, and is a graph (b) representing a result of a calculation of nuclear/cytoplasmic YAP fluorescence ratio by Y27632 and blebbistatin calculated from the confocal immunofluorescent image.

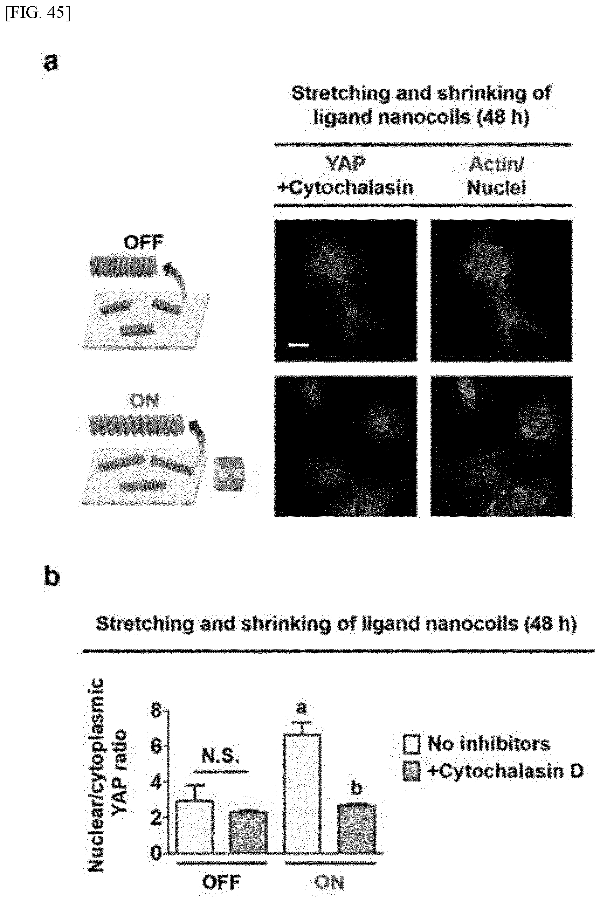

FIG. 45 is a confocal immunofluorescent image (a) of YAP, F-actin, and nuclei in stem cells cultured for 48 hours in a medium without an inhibitor and a medium with actin polymerization inhibitor (cytochalasin D) by using the nanohelix-substrate complex according to the present invention, and a scale bar represents 50 $\mu$m, and is a graph (b) representing a result of a calculation of nuclear/cytoplasmic YAP fluorescence ratio by cytochalasin D calculated from the confocal immunofluorescent image.

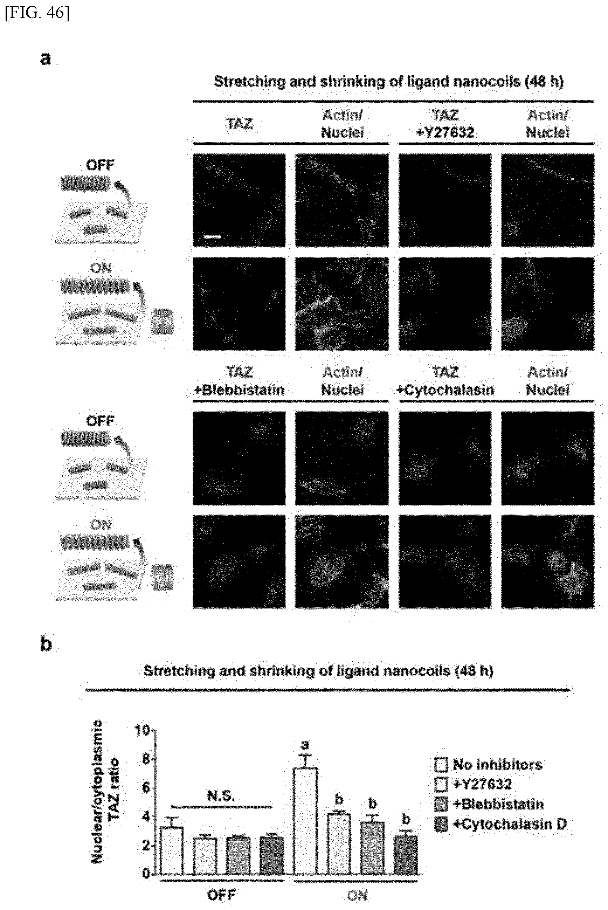

FIG. 46 is a confocal immunofluorescent image (a) of TAZ, F-actin, and nuclei in stem cells cultured for 48 hours in a medium without an inhibitor and a medium with actin polymerization inhibitor (cytochalasin D), ROCK inhibitor (Y27632), and myosin II inhibitor (blebbistatin) by using the nanohelix-substrate complex according to the present invention, and a scale bar represents 50 $\mu$m, and is a graph (b) representing a result of a calculation of nuclear/cytoplasmic TAZ fluorescence ratio by cytochalasin D, Y27632, and blebbistatin calculated from the confocal immunofluorescent image.

Referring to FIGS.44 to 46, it can be seen that mechanosensing of stem cells induced by the stretching ("ON") mode in which the magnetic field is applied in the bimodal switching involves signaling molecules, such as myosin II, rho-associated protein kinase (ROCK), and actin polymerization, which positively regulate pronounced nuclear localization of YAP/TAZ mechanotransducers.

Through this, it can be seen that the case ("ON") in which the magnetic field is applied in the bimodal switching mediates stem cell differentiation through YAP/TAZ mechanotransduction.

**Experimental Example 10**

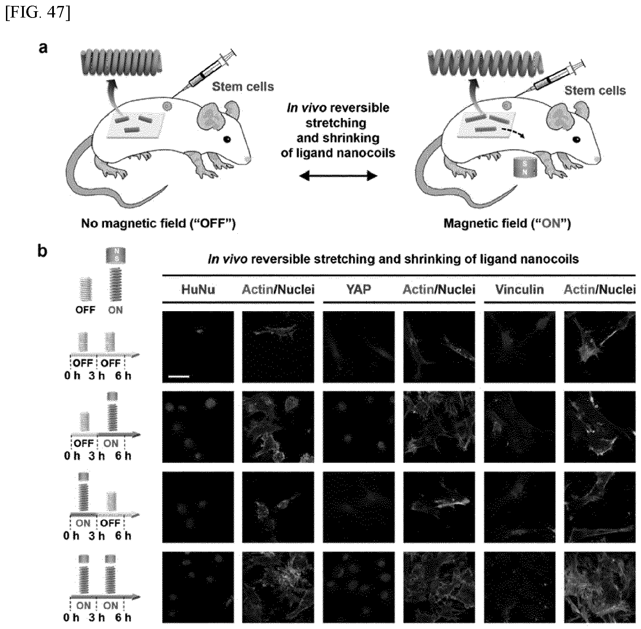

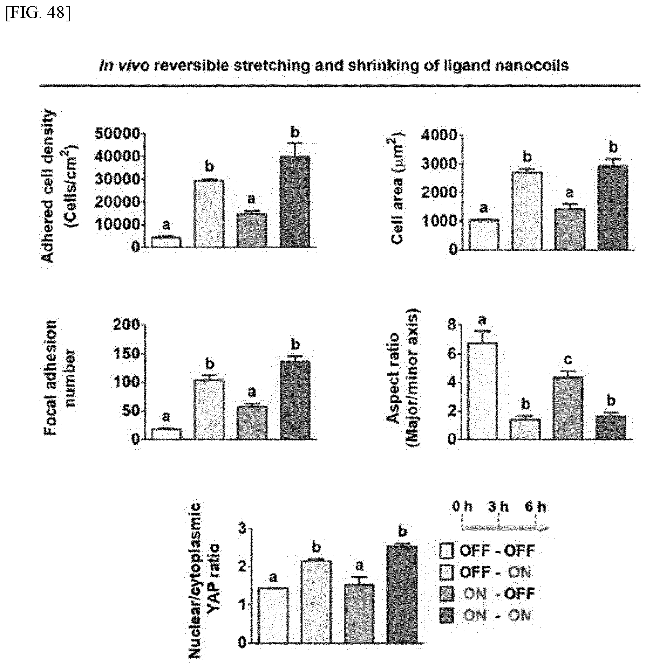

The experiment was performed to confirm the control of the adhesion and the mechanotransduction of stem cells in vivo for the stretching and the compression of the nanohelix according to the application of the magnetic field by using the nanohelix-substrate complex according to the present invention, and the result thereof is represented in FIGS. 47 and 48.

FIG. 47 is a result of an experiment for host stem cell adhesion control in vivo by using the nanohelix-substrate complex according to the present invention, and a of FIG. 47 is an immunofluorescent confocal image of human-specific nuclear antigen (HuNu), F-actin, and nucleus in stem cells in the case of including the nanohelix with different magnetic field application order 6 hours after the injection of hMSC on the subcutaneous implanted substrate, and a scale bar is 50 $\mu$m.

FIG. 48 is a graph illustrating adherent cell density, cell area, focal adhesion number, aspect ratio (major axis/minor axis ratio), and nuclear/cytoplasmic YAP fluorescence ratio calculated from the confocal immunofluorescent image of FIG. 47.

(a) of FIG. 47 is an image illustrating the experiment conducted by implementing the nanohelix-substrate complex according to the present invention into subcutaneous pockets of nude mice and then injecting hMSC.

Referring to FIGS. 47 and 48, it can be seen the injected hMSCs that had adhered to the substrate by co-localization of human-specific nuclear antigen (HuNu) and DAPI-positive nuclei in immunofluorescence of all cases in the bimodal switching. Further, immunofluorescence confirmed that in the reversible bimodal switching in vivo, the stretching ("OFF-ON" and "ON-ON" groups) group in which the magnetic field is applied stimulates significantly higher adherent density and focal adhesion of stem cells over a wider area and vinculin clustering, and YAP mechanotransduction, compared to the compression ("OFF") group in which the magnetic field is removed, which also promoted the adhesion of host immune cells over prolonged time.

**Claims**

1. A nanohelix-substrate complex for controlling behavior of regenerative cells, comprising:

a substrate;
one or more nanohelices chemically coupled to the substrate; and
one or more integrin-coupled ligand peptides chemically coupled to the nanohelix,
wherein the nanohelix is formed of a spiral nanowire and includes one or more metal elements,
the nanohelix has a length of 100 nm to 20 $\mu$m, and
the nanohelix has a length reversibly changed depending on application/non-application of a magnetic field within a range of Equation 1 below,
the regenerative cells are macrophages or stem cells, and
the nanohelix controls cell adhesion and polarization of the macrophages or controlling cell adhesion and differentiation of the stem cells,

$$[Equation\ 1]$$

$$|L_1 - L_0| > 10\ nm$$

in Equation 1, $L_1$ is a length of the nanohelix when the magnetic field is applied, and $L_0$ is a length of the nanohelix when the magnetic field is not applied.

2. The nanohelix-substrate complex of claim 1, wherein the metal element includes one or more elements among cobalt (Co), iron (Fe), and nickel (Ni),

the nanowire is provided in a form of a wire having a circular cross-section, and has a diameter of 5 nm to 100 nm, and

an average length of a spiral outer diameter of the nanohelix is 50 nm to 200 nm.

3. The nanohelix-substrate complex of claim 1, wherein a plurality of integrin-coupled ligand peptides is coupled to the nanohelix while being spaced apart from each other, and an average interval between the adjacent integrin ligands is 1 nm to 10 nm.

4. The nanohelix-substrate complex of claim 1, wherein when the magnetic field is applied, adjacent spirals of the nanohelix are spaced apart from each other, and a pitch between the adjacent spirals is 1 nm to 100 nm, and
the applied magnetic field has a size of 100 mT to 7 T.

5. The nanohelix-substrate complex of claim 1, wherein the integrin-coupled ligand peptide includes a thiolated integrin-coupled ligand peptide, and
a thiol group of the integrin-coupled ligand peptide is coupled to the spiral nanohelix by a polyethylene glycol linker.

6. The nanohelix-substrate complex of claim 1, wherein the nanohelix is coupled to the substrate by coupling carboxylate to the nanohelix, and
the surface of the substrate, which is not coupled with the nanohelix, is inactivated.

7. A method of controlling behavior of regenerative cells, the method comprising:

preparing a nanohelix by electrodepositing a solution including one or more metal elements;
coupling a carboxylate substituent to the nanohelix by mixing the nanohelix and a first suspension;
manufacturing a substrate coupled with the nanohelix by soaking a substrate of which a surface is activated in a solution containing the nanohelix to which the carboxylate is coupled;
coupling a linker to a distal end of the nanohelix by soaking the substrate coupled with the nanohelix in a solution containing a polyethylene glycol linker; and
coupling an integrin-coupled ligand peptide (RGD) to the nanohelix by mixing a second suspension containing the integrin-coupled ligand peptide (RGD) and the activated substrate coupled with the nanohelix.

8. The method of claim 7, wherein in the preparing of the nanohelix, the solution containing the metal element includes one or more elements among cobalt (Co), iron (Fe), and nickel (Ni).

9. The method of claim 7, wherein in the coupling of the carboxylate substituent, the first suspension includes an amino acid derivative containing a carboxylate substituent, and
the amino acid derivative is coupled to a surface of the nanohelix.

10. The method of claim 7, wherein in the coupling of the integrin-coupled ligand peptide, the second suspension includes thiolated integrin-coupled ligand peptide, and
the manufacturing of the substrate coupled with the nanohelix uses the substrate, of which the surface is aminated, by activating the surface of the substrate by immersing the substrate in an acid solution and then soaking the substrate, of which the surface is activated, in an aminosilane solution.

11. The method of claim 7, wherein after the coupling of the integrin-coupled ligand peptide to the nanohelix, soaking the substrate coupled with the nanohelix in a solution including a polyethylene glycol derivative and inactivating a surface of the substrate which is not coupled with the nanohelix.

12. A method of controlling behavior of regenerative cells, the method comprising:

controlling behavior of regenerative cells by treating the nanohelix-substrate complex for controlling the behavior of the regenerative cells according to any one of claims 1 to 6 with a culture medium and then applying a magnetic field in a range from 20 mT to 7 T,
wherein the nanohelix has a length reversibly changed within Equation 1 below depending on application/non-application of the magnetic field,
in the controlling of the behavior of the regenerative cells, the regenerative cells are stem cells or macrophages,
in the case where the regenerative cells are the macrophages, the controlling of the behavior of the regenerative cells includes controlling cell adhesion and polarization of the macrophages, and
in the case where the regenerative cells are the stem cells, the controlling of the behavior of the regenerative cells includes controlling cell adhesion and differentiation of the stem cells,

[Equation 1]

$$|L_1 - L_0| > 10 \text{ nm}$$

in Equation 1, $L_1$ is a length of the nanohelix when the magnetic field is applied, and $L_0$ is a length of the nanohelix when the magnetic field is not applied.

13. The method of claim 12, wherein the controlling of the adhesion and the polarization of the macrophages includes adhering and polarizing the macrophages in vivo and ex vivo by reversibly changing the length of the nanohelix depending on the application/non-application of the magnetic field to the nanohelix-substrate complex, and

the controlling of the adhesion and the differentiation of the stem cells includes adhering and differentiating the stem cells in vivo and ex vivo by reversibly changing the length of the nanohelix depending on the application/non-application of the magnetic field to the nanohelix-substrate complex.

14. The method of claim 12, wherein the controlling of the adhesion and the polarization of the macrophages promotes an inflammatory (M1) phenotype in the case where the magnetic field is not applied to the nanohelix-substrate complex, and

the controlling of the adhesion and the polarization of the macrophages promotes a regenerative and anti-inflammatory (M2) phenotype in the case where the magnetic field is applied to the nanohelix-substrate complex.

15. The method of claim 12, wherein the adhesion and mechanosensing differentiation of stem cells are degraded in the case where the magnetic field is not applied to the nanohelix-substrate complex, and the adhesion and mechanosensing differentiation of stem cells are promoted in the case where the magnetic field is applied to the nanohelix-substrate complex.

[FIG. 1]

[FIG. 2]

[FIG. 3]

[FIG. 4]

(a)

## EDS Spectrum

(b)

[FIG. 5]

[FIG. 6]

[FIG. 7]

[FIG. 8]

[FIG. 9]

[FIG. 10]

[FIG. 11]

[FIG. 12]

**a**

Bimodal switching of ligand nanohelices stretching (24 h)

**b**

Bimodal switching of ligand nanohelices stretching (24 h)

[FIG. 13]

a

b

[FIG. 14]

**a**

Bimodal switching without nanohelix or RGD ligand (24 h)

**b**

Bimodal switching without nanohelix or RGD ligand (24 h)

[FIG. 15]

a

Cyclic switching of stretching and compression of ligand nanohelices

b

Cyclic switching of stretching and compression of ligand nanohelices

[FIG. 16]

[FIG. 17]

(a) **Bimodal switching of ligand nanohelices stretching (M1 medium)**

(b) **Bimodal switching of ligand nanohelices stretching (M2 medium)**

[FIG. 18]

**a**

ROCK2/Nuclei (36 h)

**b**

[FIG. 19]

[FIG. 20]

[FIG. 21]

[FIG. 22]

**a**

*In vivo* bimodal switching of ligand nanohelices stretching

**b**

*In vivo* bimodal switching of ligand nanohelices stretching

[FIG. 23]

[FIG. 24]

**a**

Electrodeposition time-regulated tuning of nanocoil length

**b**

Template pore diameter-regulated tuning of nanocoil diameter

**c**

Precursor chemistry -regulated tuning of nanocoil element

Co nanocoil

[FIG. 25]

[FIG. 26]

[FIG. 27]

[FIG. 28]

[FIG. 29]

[FIG. 30]

[FIG. 31]

[FIG. 32]

In situ magnetic imaging of reversible nanocoil stretching and shrinking

[FIG. 33]

No magnet (Control)

[FIG. 34]

a

Stretching and shrinking of ligand nanocoils (48 h)

b

Stretching and shrinking of ligand nanocoils (48 h)

[FIG. 35]

Cyclic stretching and shrinking of ligand nanocoils

[FIG. 36]

Cyclic stretching and shrinking of ligand nanocoils

[FIG. 37]

**a**

Stretching and shrinking of
ligand nanocoils (48 h)

**b**

Stretching and shrinking of
ligand nanocoils (48 h)

[FIG. 38]

[FIG. 39]

(a) Reversible nanocoil stretching

(b) Reversible stretching and shrinking of ligand nanocoils

[FIG. 40]

[FIG. 41]

(a) Reversible nanocoil stretching

(b) Reversible stretching and shrinking of ligand nanocoils

[FIG. 42]

Reversible stretching and shrinking of ligand nanocoils

[FIG. 43]

[FIG. 44]

**a**

Stretching and shrinking of ligand nanocoils (48 h)

**b**

Stretching and shrinking of ligand nanocoils (48 h)

[FIG. 45]

**a**

Stretching and shrinking of
ligand nanocoils (48 h)

YAP
+Cytochalasin

Actin/
Nuclei

OFF

ON

**b**

Stretching and shrinking of ligand nanocoils (48 h)

□ No inhibitors
■ +Cytochalasin D

[FIG. 46]

[FIG. 47]

[FIG. 48]

*In vivo* reversible stretching and shrinking of ligand nanocoils

Europäisches
Patentamt

European
Patent Office

Office européen
des brevets

# EUROPEAN SEARCH REPORT

Application Number

EP 21 18 9669

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | MIN SUNHONG ET AL: "Remote Control of Time-Regulated Stretching of Ligand-Presenting Nanocoils In Situ Regulates the Cyclic Adhesion and Differentiation of Stem Cells", ADVANCED MATERIALS, vol. 33, no. 11, 1 March 2021 (2021-03-01) , page 2008353, XP055876490, DE ISSN: 0935-9648, DOI: 10.1002/adma.202008353 Retrieved from the Internet: URL:https://onlinelibrary.wiley.com/doi/full-xml/10.1002/adma.202008353> * abstract * * Scheme 1, figures with legends; page 8. Point 3. , Conclusion.; figures 1-3 * | 1-15 | INV. B82B3/00 B82Y5/00 B82Y15/00 C07K7/06 C12N5/0786 C12N5/0775 |
| X,P | ----- BAE GUNHYU ET AL: "Immunoregulation of Macrophages by Controlling Winding and Unwinding of Nanohelical Ligands", ADVANCED FUNCTIONAL MATERIALS, vol. 31, no. 37, 18 June 2021 (2021-06-18) , page 2103409, XP055876487, DE ISSN: 1616-301X, DOI: 10.1002/adfm.202103409 Retrieved from the Internet: URL:https://onlinelibrary.wiley.com/doi/full-xml/10.1002/adfm.202103409> * abstract * * Scheme 1; figures with legends; Section 2 Results and discussion, pages 3-13; page 14, Section 3: Conclusion.; figures 1-6 * ----- -/-- | 1-15 | TECHNICAL FIELDS SEARCHED (IPC) B82B B82Y C12N C07K |

The present search report has been drawn up for all claims

| Place of search | Date of completion of the search | Examiner |
|---|---|---|
| Munich | 11 January 2022 | Bretherick, James |

EPO FORM 1503 03.82 (P04C01)

**page 1 of 3**

Europäisches Patentamt

European Patent Office

Office européen des brevets

## EUROPEAN SEARCH REPORT

Application Number

EP 21 18 9669

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHOI HYOJUN ET AL: "In Situ Magnetic Control of Macroscale Nanoligand Density Regulates the Adhesion and Differentiation of Stem Cells", ADVANCED FUNCTIONAL MATERIALS, vol. 30, no. 35, 1 August 2020 (2020-08-01), page 2001446, XP055814644, DE ISSN: 1616-301X, DOI: 10.1002/adfm.202001446 * Abstract; Scheme 1 and legend ; figures and associated legends. Page 11, point 3, conclusion.; figures 1-7 * | 1-15 | |
| A | MIN SUNHONG ET AL: "In Situ Magnetic Control of Macroscale Nanoligand Density Regulates the Adhesion and Differentiation of Stem Cells", ADVANCED MATERIALS, vol. 32, no. 40, 20 August 2020 (2020-08-20), page 2004300, XP055845768, DE ISSN: 0935-9648, DOI: 10.1002/adma.202004300 Retrieved from the Internet: URL:https://onlinelibrary.wiley.com/doi/full-xml/10.1002/adma.202004300> * abstract * * Scheme 1 and legend, figures and legends.; figures 1-3 * | 1-15 | TECHNICAL FIELDS SEARCHED (IPC) |

-/--

The present search report has been drawn up for all claims

| Place of search | Date of completion of the search | Examiner |
|---|---|---|
| Munich | 11 January 2022 | Bretherick, James |

EPO FORM 1503 03.82 (P04C01)

page 2 of 3

Europäisches
Patentamt

European
Patent Office

Office européen
des brevets

# EUROPEAN SEARCH REPORT

Application Number

EP 21 18 9669

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KHATUA CHANDRA ET AL: "In Situ Magnetic Control of Macroscale Nanoligand Density Regulates the Adhesion and Differentiation of Stem Cells", NANO LETTERS, vol. 20, no. 6, 10 June 2020 (2020-06-10), pages 4188-4196, XP055817288, US ISSN: 1530-6984, DOI: 10.1021/acs.nanolett.0c00559 Retrieved from the Internet: URL:https://pubs.acs.org/doi/pdf/10.1021/acs.nanolett.0c00559> * abstract * * Scheme 1, Figures and legends.; figures 1-4 * ----- | 1-15 | |
| A | US 2018/327919 A1 (KIM YOUNG KEUN [KR] ET AL) 15 November 2018 (2018-11-15) * paragraph [0019] – paragraph [0026] * * paragraph [0027] – paragraph [0057]; claims 1-10 * ----- | 1-15 | TECHNICAL FIELDS SEARCHED (IPC) |

The present search report has been drawn up for all claims

| Place of search | Date of completion of the search | Examiner |
|---|---|---|
| Munich | 11 January 2022 | Bretherick, James |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 21 18 9669

This annex lists the patent family members relating to the patent documents cited in the above-mentioned European search report.
The members are as contained in the European Patent Office EDP file on
The European Patent Office is in no way liable for these particulars which are merely given for the purpose of information.

11-01-2022

| Patent document cited in search report | | Publication date | Patent family member(s) | | Publication date |
|---|---|---|---|---|---|
| US 2018327919 | A1 | 15-11-2018 | JP | 6518307 B2 | 22-05-2019 |
| | | | JP | 2018193607 A | 06-12-2018 |
| | | | KR | 101916588 B1 | 07-11-2018 |
| | | | US | 2018327919 A1 | 15-11-2018 |

EPO FORM P0459

For more details about this annex : see Official Journal of the European Patent Office, No. 12/82

**EP 3 984 946 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101916588